# EUROPÄISCHE PATENTSCHRIFT

(11) **EP 1 576 059 B1**
(45) Veröffentlichungstag und Bekanntmachung des Hinweises auf die Patenterteilung: **27.06.2012**
(21) Anmeldenummer: 03799491.0
(22) Anmeldetag: 18.12.2003
(51) Int. Cl.: C09B 11/04, C07H 21/00, G01N 33/533, G01N 33/58, C12Q 1/68

(54) **CARBOXAMID-SUBSTITUIERTE FARBSTOFFE FÜR ANALYTISCHE ANWENDUNGEN**
CARBOXAMIDE-SUBSTITUTED DYES FOR ANALYTICAL APPLICATIONS
COLORANTS SUBSTITUES PAR CARBOXAMIDE POUR APPLICATIONS ANALYTIQUES

(30) Priorität: 18.12.2002 DE 10259374
(43) Veröffentlichungstag der Anmeldung: 21.09.2005
(73) Patentinhaber: ATTO-TEC GmbH, 57076 Siegen (DE)
(72) Erfinder: ARDEN-JACOB, Jutta, 90513 Zirndorf (DE); DREXHAGE, Karl-Heinz, 57076 Siegen (DE); HAMERS-SCHNEIDER, Monika, 57258 Freudenberg (DE); KEMNITZER, Norbert, 57250 Netphen (DE); ZILLES, Alexander, 55490 Mengerschied (DE)
(74) Vertreter: Weiss, Wolfgang
(86) Internationale Anmeldenummer: PCT/EP2003/014534
(87) Internationale Veröffentlichungsnummer: WO 2004/055117

(56) Entgegenhaltungen:
- EP-A- 0 167 998
- EP-A- 0 347 697
- WO-A-00/64987
- WO-A-00/64988
- WO-A-02/055512
- WO-A-03/098618
- GB-A- 1 407 331
- GB-A- 1 445 989

## Beschreibung

Die vorliegende Erfindung bezieht sich auf Carboxamid-substituierte Farbstoffe, deren Herstellung und auf die Verwendung solcher Farbstoffe als Markierungsgruppen in der Analytik.

Farbstoffe aus der Klasse der Xanthen- und Triphenylmethanfarbstoffe sowie verwandte Derivate werden aufgrund ihrer sehr guten spektralen Eigenschaften bevorzugt in der chemischen, biologischen und medizinischen Analytik als Markierungsgruppen eingesetzt (J. Slavik, Fluorescent Probes in Cellular and Molecular Biology, CRC Press, Boca Raton, Ann Arbor, London, Tokyo, 1994). Farbstoffe aus verwandten Klassen der Carbopyronine und Amidopyrylium-Farbstoffe werden beschrieben in WO 00/64986 und WO 00/64987. Dabei spielen vor allem Farbstoffe mit einer sehr hohen Fluoreszenzquantenausbeute eine wichtige Rolle, da über die Fluoreszenz ein sehr empfindlicher Nachweis des markierten Analyten möglich ist. Aber auch nicht-fluoreszierende Derivate gewinnen zunehmend als Quencher in speziellen Verfahren Bedeutung.

Zur Verwendung als Markierungsgruppen in Nachweisverfahren für Analyten ist neben einer einfachen und zuverlässigen Nachweisbarkeit eine gute Löslichkeit in verschiedenen Lösungsmitteln, insbesondere in wässrigen Systemen, erforderlich. Weiterhin sollten derartige Verbindungen einfach und kostengünstig herzustellen sein und eine hohe Stabilität und Lagerfähigkeit aufweisen.

Viele Farbstoffe aus den oben genannten Farbstoffklassen besitzen eine Carboxylgruppe, die aufgrund ihrer Position und der Molekülstruktur in Abhängigkeit von Lösungsmittelumgebung und pH-Wert, z.B. bereits in neutraler oder schwach basischer Lösung, zur Bildung eines farblosen Lactons Anlass gibt. Vgl. dazu etwa K.H. Drexhage, Structure and Properties of Laser Dyes, in: F.P. Schäfer, Topics in Applied Physics, Vol. 1, Dye Lasers, Springer-Verlag, Berlin, Heidelberg, New York, 1973.

Bei der üblichen kovalenten Kopplung, z.B. über einen Aktivester, einer derartigen Carboxylgruppe an die primäre Aminogruppe eines Analyten (Peptid etc.) entsteht ein Säureamid, das sich sofort gemäß dem folgenden Schema zu einem Lactam umlagert:

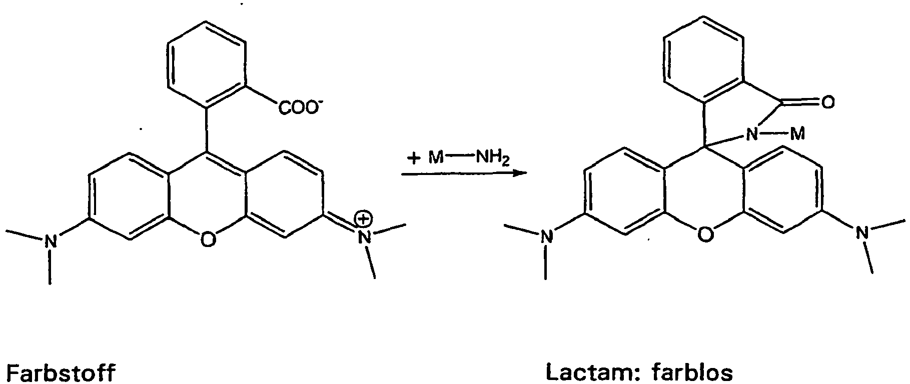

Dieses ist jedoch unter physiologischen Bedingungen farblos, so dass die Farbstoffmarkierung fehlschlägt. Das heißt, der markierte Analyt kann nicht durch die Absorption und/oder Fluoreszenz der Markierungsgruppe nachgewiesen werden. Viele bekannte und leicht zugängliche Farbstoffe (z.B. Rhodamine) scheiden deshalb für die Verwendung als Marker-Farbstoffe aus. Diese Schwierigkeit ließ sich bisher nur dadurch umgehen, dass man zusätzliche Kopplungsgruppen in das Farbstoffmolekül eingeführt hat. Die dazu erforderlichen Vorprodukte sind jedoch meist schwer zugänglich und erfordern aufwändige Syntheseschritte.

WO 02/055512 offenbart die Herstellung von Amid-Derivaten von Fluorescein-Farbstoffen, wobei die Carbonsäuregruppe zunächst bei höheren Temperaturen zu einem Aktivester umgesetzt und dann der Aktivester mit einem sekundären Amin in einem wässrigen Lösungsmittelgemisch zur Reaktion gebracht wird. Das Verfahren gemäß WO 02/055512 ist nur anwendbar auf Fluorescein und seine Derivate und kann nicht auf andere Farbstoffklassen übertragen werden. Insbesondere sind die offenbarten Verfahrensbedingungen, wie Temperatur und verwendete Lösungsmittel, nicht auf Amino- oder/und Iminogruppenenthaltende Verbindungen, wie beispielsweise Rhodamine, anwendbar. Zum einen führen die bei der Aktivesterbildung eingestellten Temperaturen zu Neben- und Zersetzungsprodukten, zum anderen kann das bei der Umsetzung des Aktivesters mit einem Amin als Lösungsmittel verwendete Wasser mit dem Aktivester reagieren, was wiederum unerwünschte Produkte zur Folge hat.

WO 00/64988 A1 offenbart aminosubstituierte Xanthen-Farbstoffe, welche Lumineszenz-quenchende Eigenschaften besitzen und u.a. zur Markierung von Biomolekülen eingesetzt werden können.

EP 0 347 697 A2 offenbart basische Rhodamin-Farbstoffe, welche zwei über eine tertiäre Aminogruppe miteinander verbundene Rhodaminmoleküle umfassen und zum Färben von Papierstoffen verwendet werden können.

EP 0167 998 A2 offenbart Rhodamin-Farbstoffe mit Carbonsäureamidgruppe, welche insbesondere zum Färben von Papierstoffen eingesetzt werden können.

GB 1 407 331 und GB 1 445 989 offenbaren jeweils verbrückte Triphenylmethan-Farbstoffe, welche zum Färben von synthetischen polymeren Materialien verwendet werden können.

WO 03/098618A1 offenbart optische Speichermedien, welche u.a. Farbstoffe mit Xanthen-Substruktur umfassen.

Eine Aufgabe der vorliegenden Erfindung besteht daher darin, Lacton- bzw. Lactam-bildende Farbstoffe mit unterschiedlichen funktionellen Gruppen auf einfache Weise so zu modifizieren, dass sie als Marker in verschiedenen Bereichen Verwendung finden können.

Gelöst wird diese Aufgabe durch Bereitstellung eines Carboxamid-substituierten Farbstoffs der allgemeinen Formel (I) 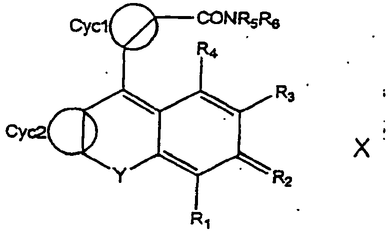 wobei
Y = Sauerstoff, Schwefel, Selen, CRₐR_{b}, NR_{c}, eine direkte Verknüpfung oder -R₁₄ und -R₁₅ darstellt;
R₁, R₃, R₄ unabhängig Wasserstoff, Halogen (wie Fluor, Chlor, Brom, Jod), -O^{e}, eine Hydroxygruppe, Thiolgruppe, Aminogruppe, Ammoniumgruppe, sulfongruppe, Phosphogruppe, Nitrogruppe, Carbonylgruppe (z.B. Keto- oder Aldehydgruppe), Carboxygruppe, ein Carbonsäurederivat (wie Carboxylat, Ester, Halogenid, Amid, Anhydrid), Nitrilgruppe, Isonitrilgruppe, Cyanatgruppe, Isocyanatgruppe, Thiocyanatgruppe, Isothiocyanatgruppe oder einen geradkettigen, verzweigten oder cyclischen gesättigten oder ungesättigten Kohlenwasserstoffrest mit bis zu 40 Kohlenstoffatomen darstellen;
Rₐ. R_{b}, R_{c} und R₁₄, R₁₅ unabhängig die gleichen Bedeutungen haben, wie es für R₁, R₃, R₄ definiert ist;
R₂=O; 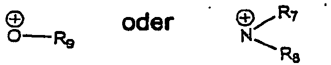 darstellt, wobei
R₇, R₈, R₉ unabhängig Wasserstoff oder einen geradkettigen, verzweigten oder cyclischen gesättigten Kohlenwasserstoffrest mit bis zu 40 Kohlenstoffatomen darstellen; oder
R₁ zusammen mit R₂ 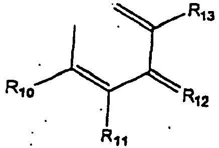 darstellt, wobei
R₁₀, R₁₁, R₁₃ die gleichen Bedeutungen haben, wie es für R₁, R₃, R₄ definiert ist;
R₁₂=O; 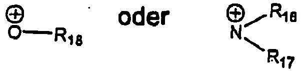
darstellt, wobei
R₁₆ R_{17,} R₁₈ die gleichen Bedeutungen aufweisen, wie es für R_{7,} R₈, R₉ definiert ist;
R₅ einen geradkettigen, verzweigten oder cyclischen gesättigten oder ungesättigten Kohlenwasserstoffrest mit bis zu 40 Kohlenstoffatomen darstellt, welcher Heteroatome enthalten kann und unsubstituiert oder substituiert sein kann;
R₆ eine geradkettige oder verzweigte gesättigte oder ungesättigte Kohlenwasserstoffgruppe mit bis zu 40 Kohlenstoffatomen darstellt, welche Heteroatome enthalten kann und einen Substituenten ausgewählt aus einer Carboxylgruppe, Cyanatgruppe, Iminogruppe, Iminiumgruppe, Isocyanatgruppe, Isothiocyanatgruppe, Nitrogruppe, Phosphogruppe, Sulfogruppe, Thiocyanatgruppe, Thioethergruppe, Thiolgruppe oder einem Carbonsäurederivat umfasst;
Cyc1 einen organischen Rest darstellt, der ein Ringsystem umfasst, ausgewählt aus aromatischen, heteroaromatischen, chinoiden und cycloaliphatischen Ringen;
Cyc2 einen organischen Rest darstellt, der ein Ringsystem umfasst, ausgewählt aus aromatischen, heteroaromatischen, chinoiden und cycloaliphatischen Ringen;
wobei jeder der Reste in der Verbindung gemäß Formel (I) mit einem oder mehreren benachbarten Resten ein Ringsystem bilden kann; und X, wenn es zum Ladungsausgleich erforderlich ist, ein oder mehrere ein- oder mehrwertige Anionen darstellt; mit der Maßgabe, dass in der allgemeinen Formel (I) nicht gleichzeitig
   - Y = Sauerstoff
   - Cyc1 = Phenyl oder substituiertes Phenyl,
   - Cyc2 = Hydroxy-, Ether- oder Ester-substituiertes Phenyl
      und
   - R₂=O
darstellen.

Vorzugsweise sind die Substituenten in der allgemeinen Formel (I) so gewählt, dass nicht gleichzeitig
- Y = Sauerstoff
- Cyc1 = Phenyl oder substituiertes Phenyl,
- Cyc2 = Phenyl oder substituiertes Phenyl
   und
- R₂ = O oder 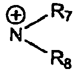
wenn R₇ oder/und R₈ kein Ringsystem mit benachbarten Substituenten bildet,
darstellen.

Überraschenderweise gelang es, unterschiedlich funktionalisierte Farbstoffe gemäß der allgemeinen Formel (I) bereitzustellen, welche sehr gute spektrale Eigenschaften, wie Lage der Absorptions- und Fluoreszenzbanden, hohe Extinktionskoeffizienten sowie hohe Fluoreszenzquantenausbeuten und Stabilitäten aufweisen. Der Nachteil der Lacton- bzw. Lactambildung, der bei gewöhnlich verwendeten Carboxylsubstituierten Farbstoffen auftritt, wird durch die Generierung von sekundären Amidgruppen verhindert.

Es hat sich gezeigt, dass außerdem durch die Einführung von verschiedenen Resten an der Amid-Gruppe die Eigenschaften des Farbstoffs gesteuert werden können. So kann beispielsweise die Lipophilie des Farbstoffs durch Einführung von langen Alkylketten als Reste an der Amidgruppe erhöht werden. Andererseits kann durch Einführung von z.B. Zucker-Resten oder anderen polaren Gruppen die Hydrophilie des Farbstoffs erhöht werden. Dadurch lassen sich auf einfache Weise die Eigenschaften, wie das Löslichkeitsverhalten, modulieren. Des Weiteren können an der Amidgruppe verschiedene Linker eingebaut werden, um den Farbstoff über diese Linker z.B. an einen nachzuweisenden Analyten, wie ein Peptid oder ähnliches, koppeln oder konjugieren zu können.

Erfindungsgemäß umfassen die Kohlenwasserstoffgruppen Alkylgruppen, Alkenylgruppen, Alkinylgruppen, Cycloalkylgruppen, Arylgruppen und Heteroarylgruppen. Diese Gruppen können Heteroatome, wie Sauerstoff, Schwefel oder/und Stickstoff, enthalten. Des Weiteren können an diese Gruppen weitere Substituenten gebunden sein, die vorzugsweise ausgewählt sind aus Halogen (wie Fluor, Chlor, Brom, Jod), -O^{e}, einen. Hydroxygruppe, Thiolgruppe, Aminogruppe, Ammoniumgruppe, Sulfogruppe, Phosphogruppe, Nitrogruppe, Carbonylgruppe (z.B. Keto-oder Aldehydgruppe), Carboxygruppe, einem Carbonsäurederivat (z.B. Carboxylat, Ester, Carbonsäurehalogenid, -amid, -anhydrid), einer Nitrilgruppe, Isonitrilgruppe, Cyanatgruppe, Isocyanatgruppe, Thiocyanatgruppe, Isothiocyanatgruppe, Iminogruppe,Iminiumgruppe, Alkoxygruppe, Ethergruppe, Thioethergruppe und geradkettigen, verzweigten oder cyclischen gesättigten oder ungesättigten Kohlenwasserstoffgruppen mit bis zu 40 Kohlenstoffatomen.

Der Ausdruck "Alkylgruppen" umfasst dabei geradkettige oder verzweigte C₁-C₄₀-Alkylgruppen, vorzugsweise C₂-C₃₅-Alkylgruppen, mehr bevorzugt C₅-C₃₀-Alkylgruppen, noch mehr bevorzugt C₈-C₂₀-Alkylgruppen. Beispielsweise sind die Alkylgruppen ausgewählt aus Methyl, Ethyl und geradkettigen oder verzweigten Propyl-, Butyl-, Hexyl-, Decyl-, Dodecyl- und Octadecylgruppen.

Die "Alkenylgruppen" umfassen geradkettige oder verzweigte C₂-C₄₀-Alkenylgruppen mit ein oder mehreren Doppelbindungen an beliebiger Stelle des Kohlenwasserstoffrestes. Je nach Kettenlänge sind 1-10, 2-8 oder 4-6 Doppelbindungen pro Rest bevorzugt. Die Alkenylreste weisen 2-40, vorzugsweise 4-35, mehr bevorzugt 8-25, noch mehr bevorzugt 15-20 Kohlenstoffatome auf. Beispiele für geeignete Alkenylgruppen sind Ethenyl, Propenyl, Butenyl, Hexenyl, Decenyl, Dodecenyl und Octadecenyl.

In den geradkettigen oder verzweigten Alkinylresten liegen entsprechend ein oder mehrere Dreifachbindungen an beliebiger Position des Kohlenwasserstoffrestes vor. Je nach Kettenlänge sind 1-10, 2-8 oder 4-6 Dreifachbindungen pro Rest bevorzugt. Die Alkinylreste weisen 2-40, vorzugsweise 4-35, mehr bevorzugt 8-25, noch mehr bevorzugt 15-20 Kohlenstoffatome auf. Beispiele für geeignete Alkinylgruppen sind Ethinyl, Propinyl, Butinyl, Hexinyl, Decinyl, Dodecinyl und Octadecinyl.

Die Cycloalkylgruppen können gesättigt oder ungesättigt sein, d.h. gegebenenfalls ein oder mehrere Doppel- oder/und Dreifachbindungen aufweisen. Bevorzugt sind C₃-C₄₀-Cycloalkylgruppen, mehr bevorzugt C₃-C₂₀-Cycloalkylgruppen, noch mehr bevorzugt C₄-C₁₂ Cycloalkylgruppen, noch mehr bevorzugt C₅- oder C₆-Cycloalkylgruppen. Insbesondere sind geeignete Cycloalkylgruppen ausgewählt aus Cyclopropyl, Cyclobutyl, Cyclopentyl, Cyclopentenyl, Cyclohexyl, Cyclohexenyl, Cyclohexadienyl und Cyclooctyl. Des Weiteren können die C₃-C₄₀-Cycloalkylgruppen überbrückt sein und bicyclische bzw. polycyclische Verbindungen bilden. Beispiele für polycyclische Reste sind Norbornan-, Norbornen- und Bicyclo [3.2.2]octyl-Reste bzw. die jeweiligen substituierten Derivate davon. Die Cycloalkylgruppen schließen außerdem aliphatische Heterocyclen ein, wie z.B. Tetrahydropyrrol-, Piperidin-, Dioxan-, Tetrahydrofurangruppen.

Bevorzugte Arylreste sind C₃-C₄₀-Arylgruppen, mehr bevorzugt C₆-C₂₀-Arylgruppen und C₁₀-C₁₄-Arylgruppen. Beispiele für geeignete Arylgruppen sind Phenyl-, Naphthyl-, Anthracen- und Phenanthrengruppen.

Die Heteroarylgruppen stellen aromatische Reste mit 3-40 Kohlenstoffatomen dar, die ein oder mehrere Heteroatome aufweisen, wobei die Heteroatome ausgewählt sind aus Sauerstoff, Schwefel und Stickstoff. Vorzugsweise enthalten die Heteroarylgruppen 1-10, bevorzugt 2-6, mehr bevorzugt 3-5 Heteroatome. Die Heteroarylgruppen enthalten 3-40, insbesondere 3-18, mehr bevorzugt 4-14, noch mehr bevorzugt 5-8 Kohlenstoffatome. Beispiele für geeignete Heteroarylgruppen sind Pyrrol-, Pyridin-, Pyrimidin-, Indol-, Furan-, Thiophen- und Thiazolgruppen. Kohlenwasserstoffgruppen, die Heteroatome enthalten, sind z.B. Heteroatom-enthaltende Alkylgruppen, z.B. geradkettige oder verzweigte C₁-C₄₀-Alkoxygruppen, vorzugsweise C₁-C₁₈-Alkoxygruppen, mehr bevorzugt C₂-C₁₄-Alkoxygruppen, noch mehr bevorzugt C₆-C₁₂-Alkoxygruppen. Beispiele für geeignete Alkoxygruppen sind Methoxy, Ethoxy, geradkettiges oder verzweigtes Propoxy, Butoxy, Decoxy und Undecoxy.

Beispiele für Heteroatom-enthaltende Arylgruppen sind Aryloxygruppen, wie beispielsweise Phenoxy und Naphthoxy.

Heteroatom-enthaltende Kohlenwasserstoffreste gemäß der vorliegenden Erfindung umfassen des Weiteren z.B. Carbonylgruppen (wie Keto- oder Aldehydgruppen), Carbonsäuren, Carbonsäurederivate (wie Halogenide, Ester, Amide, Anhydride, Caboxylate), Ether, Thioether und Alkoxycarbonylgruppen, welche entsprechend 1-40 Kohlenstoffatome, bevorzugt 2-35, mehr bevorzugt 5-30 und auch bevorzugt 8-20 Kohlenstoffatome enthalten.

Erfindungsgemäß können auch Cyc1 und Cyc2 weiter substituiert sein, wobei die Substituenten vorzugsweise ausgewählt sind aus Halogen (wie Fluor, Chlor, Brom, Jod), -O^{e}, einer Hydroxygruppe, Thiolgruppe, Aminogruppe, Ammoniumgruppe, Sulfogruppe, Phosphogruppe, Nitrogruppe, Carbonylgruppe (z.B. Keto- oder Aldehydgruppe), Carboxygruppe, einem Carbonsäurederivat (wie Carboxylat, Ester, Halogenid, Amid, Anhydrid), einer Nitrilgruppe, Isonitrilgruppe, Cyanatgruppe, Thiocyanatgruppe, lsocyanatgruppe, Isothiocyanatgruppe, Iminogruppe, Iminiumgruppe, oder einer wie oben definierten geradkettigen, verzweigten oder cyclischen ungesättigten oder gesättigten Kohlenwasserstoffgruppe mit gegebenenfalls ein oder mehreren Heteroatomen.

Jeder der in dem erfindungsgemäßen Carboxamid-substituierten Farbstoff der allgemeinen Formel (I) enthaltenen Reste kann gegebenenfalls mit einem oder mehreren benachbarten Resten ein Ringsystem ausbilden. Das Ringsystem enthält vorzugsweise 5- oder/und 6-gliedrige Ringe. Bevorzugt bilden R₁ mit R₈ oder/und R₃ mit R₇ oder R₁₁ mit R₁₇ oder/und R₁₃ mit R₁₆ ein solches Ringsystem aus.

Die gegebenenfalls zum Ladungsausgleich erforderlichen Anionen können ausgewählt sein aus anorganischen oder/und organischen Anionen, wie z.B. Halogeniden, Sulfaten, Carbonaten, Phosphaten, Sulfiten, Sulfiden, Hydroxiden, Alkoxiden, Carboxylaten, Nitraten, Nitriten etc.

Vorzugsweise handelt es sich bei den erfindungsgemäßen Carboxamid-substituierten Farbstoffen der allgemeinen Formel (I) um Farbstoffe, in welchen Cyc2 in Formel (I) einen Stickstoff-enthaltenden Heterocyclus oder ein Ringsystem darstellt, welches mit mindestens einer Aminogruppe substituiert ist. Dabei ist weiterhin bevorzugt, dass R₂ 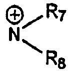 darstellt,
oder dass R₂ mit R₁ zusammen 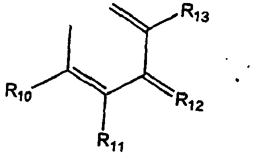 bildet,
wobei es besonders bevorzugt ist, wenn R₁₂ 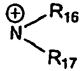 bedeutet.

In einer bevorzugten Ausführungsform der vorliegenden Erfindung weist Cyc2 in Formel (I) eine der Strukturen (A), (B), (C), (D), (E), (F), (G) (H) oder (J) auf: 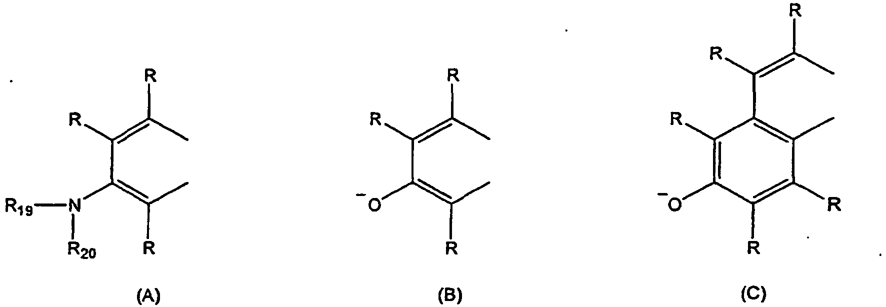 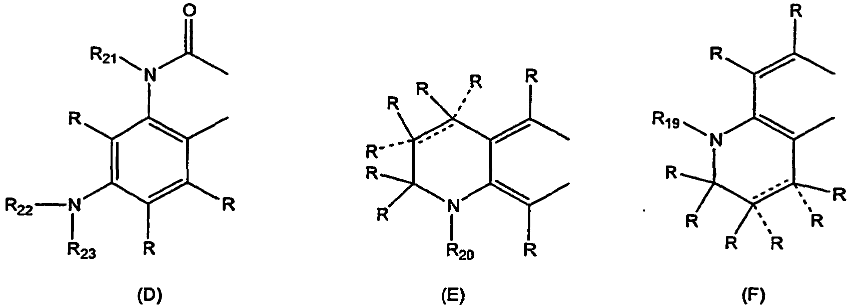 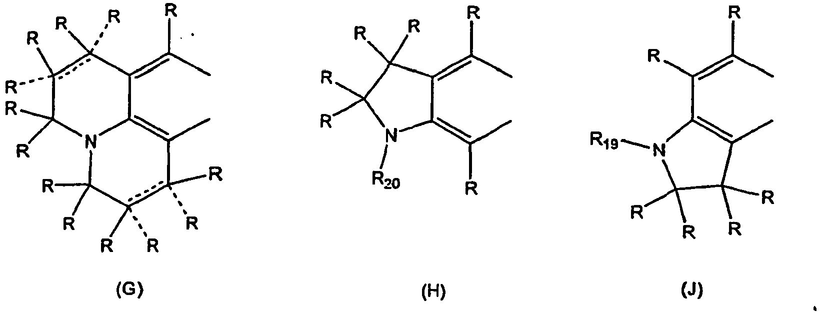
wobei R jeweils unabhängig wie R₁, R₃, R₄ definiert ist,
R₁₉, R₂₀ und R₂₂, R₂₃ unabhängig wie R₇, R₈ definiert sind; und
R₂₁ wie R₇ definiert ist und die gestrichelten Linien gegebenenfalls Doppelbindungen bedeuten, bei deren Vorhandensein die über eine gestrichelte Linie gebundenen Reste fehlen. Die Substrukturen (A) bis (J) sind so an die übrige Ringstruktur der Formel (I) kondensiert, dass die Verknüpfungsstellen durch Einfach- oder Doppelbindungen miteinander verbunden sind. Bevorzugt liegen zwischen den Verknüpfungsstellen Doppelbindungen vor.

In den Strukturen (A) bis (J) können benachbarte Substituenten gegebenenfalls weitere Ringsysteme wie oben definiert ausbilden. Dabei bilden bevorzugt die Reste R_{19,} R₂₀ und R₂₂' R₂₃ mit den jeweilig benachbarten Resten R Ringsysteme, die 5- oder/und 6-gliedrige Ringe umfassen, welche gegebenenfalls weitere Heteroatome enthalten können. Es ist besonders bevorzugt, dass Cyc2 eine der Strukturen (A), (D), (E), (F), (G),(H) oder (J) aufweist.

Die ausgehend von R₇ bzw. R₈ mit benachbarten Substituenten gebildeten Ringsysteme führen vorzugsweise zu folgenden Systemen (K), (L), (M), (N) oder (O): 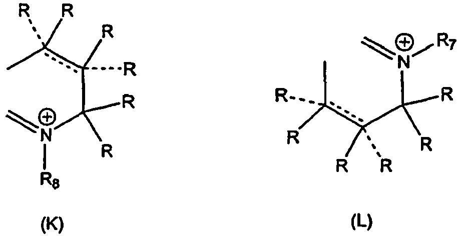 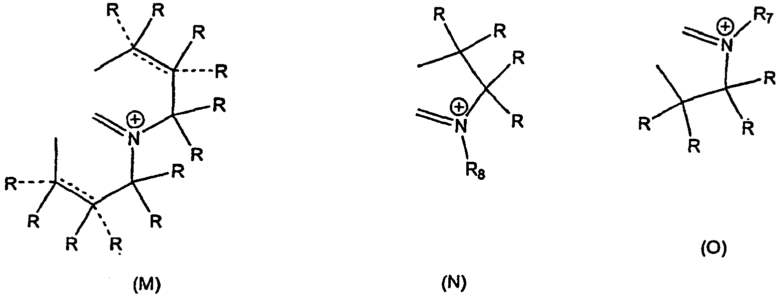
wobei R jeweils unabhängig wie R₁, R₃, R₄ definiert ist,
und die gestrichelten Linien gegebenenfalls Doppelbindungen bedeuten, bei deren Vorhandensein die über eine gestrichelte Linie gebundenen Reste fehlen. Auch in den Substrukturen (K) bis (O) können benachbarte Substituenten weitere Ringsyssteme wie oben definiert ausbilden.

In einer weiteren bevorzugten Ausführungsform der vorliegenden Erfindung stellt R₂ in Formel (I) zusammen mit R₁ 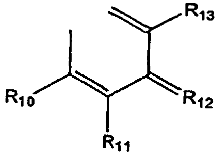 dar. Besonders bevorzugt ist dabei die Gruppe der Farbstoffe, bei denen R₁₂ Sauerstoff oder 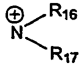 darstellt.

Bevorzugte Verbindungsklassen der vorliegenden Erfindung gemäß Formel (I) sind in den Formeln (la) bis (Ih) dargestellt: 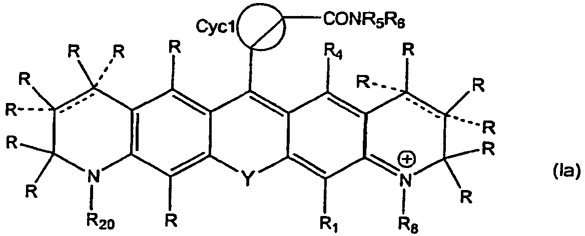 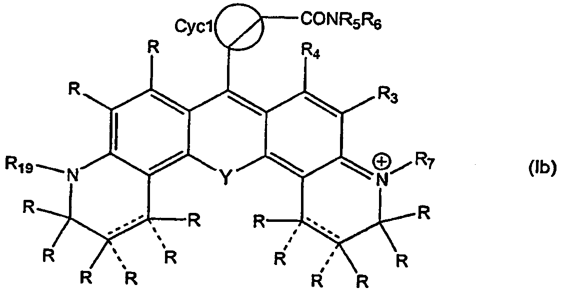 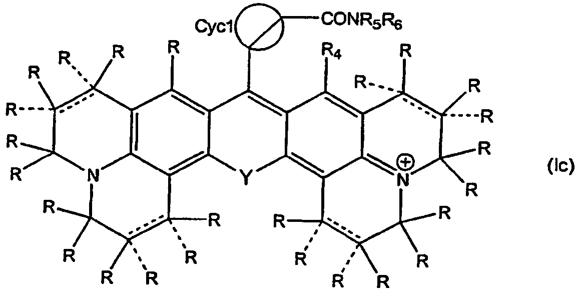 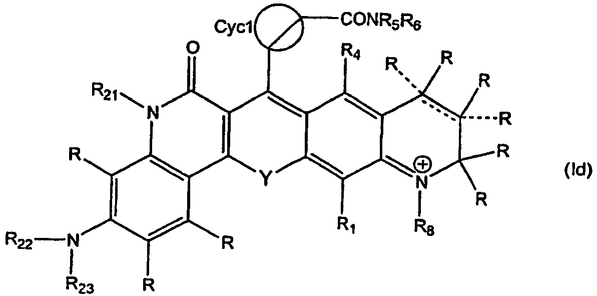 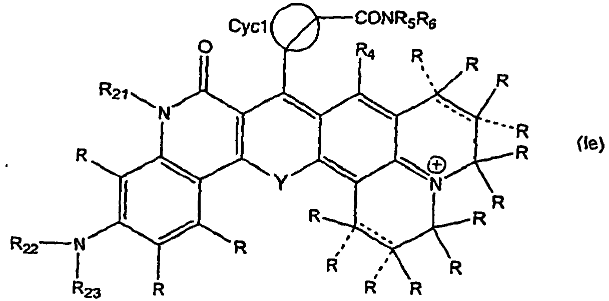 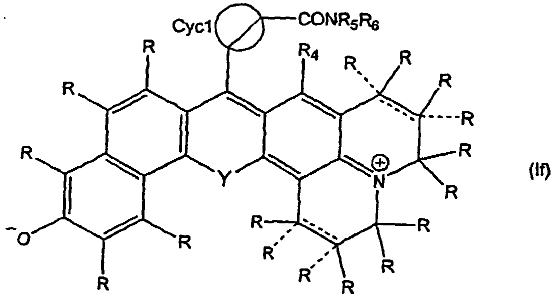 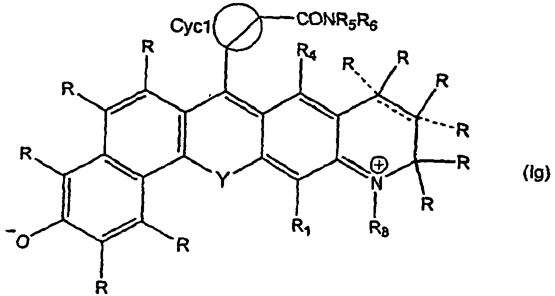 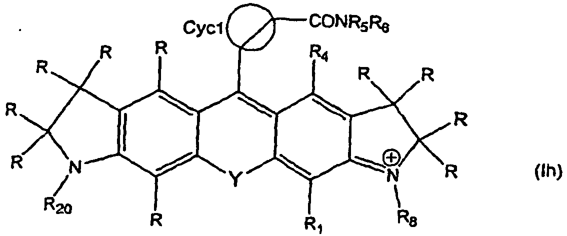 wobei die Bedeutungen der Reste wie oben definiert sind.

In den erfindungsgemäßen Verbindungen stellt Cyc1 einen gegebenenfalls substituierten organischen Rest dar, der ein Ringsystem umfasst, das vorzugsweise ausgewählt ist aus substituiertem und unsubstituiertem Phenyl, Naphthyl, Pyridyl und Cyclohexyl.

In den Carboxamid-substituierten Farbstoffen der allgemeinen Formel (I) der vorliegenden Erfindung können die Reste R₅ oder/und R₆ der Carboxamidgruppe einen Substituenten aufweisen, der geeignet ist, um an andere Moleküle zu koppeln. Besonders bevorzugt kann über einen solchen Substituenten eine kovalente Kopplung erreicht werden. Geeignete Substituenten an R₅ oder/und R₆ sind eine Carboxylgruppe, Aminogruppe, Hydroxylgruppe, Thiolgruppe, Cyanogruppe, Halogengruppe oder/und Gruppen mit ungesättigten Einheiten, wie z.B. Doppel- und Dreifachbindungen. Mittels dieser Funktionalität können die erfindungsgemäßen Farbstoffe beispielsweise an Biomoleküle oder Träger gebunden werden.

In einer bevorzugten Ausführungsform stellt mindestens einer der Reste R₅ und R₆ eine Carboxy-substituierte Alkylgruppe dar, wobei der nicht mit einer Carboxylgruppe substituierte Rest vorzugsweise eine Alkylgruppe darstellt.

Durch Variation der Reste in den erfindungsgemäßen Carboxamid-substituierten Farbstoffen der Formel (I), insbesondere der leicht einführbaren Reste R₅ oder/und R⁶, können außerdem die Eigenschaften der resultierenden Farbstoffmoleküle hinsichtlich Löslichkeit und Fluoreszenzeigenschaften auf einfache Weise eingestellt und modifiziert werden.

Tragen R₅ oder/und R₆ z.B. längere Alkylketten, so erhöht sich der lipophile Charakter, und die Verbindung ist in unpolaren Medien und Membranen löslich und kann so beispielsweise zum Nachweis von Membraneigenschaften oder zu molekularen Abstandsmessungen eingesetzt werden.

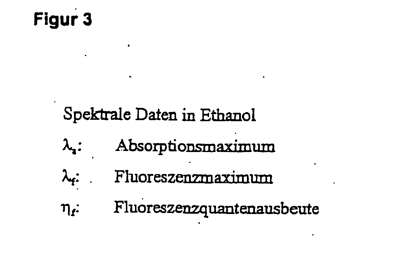

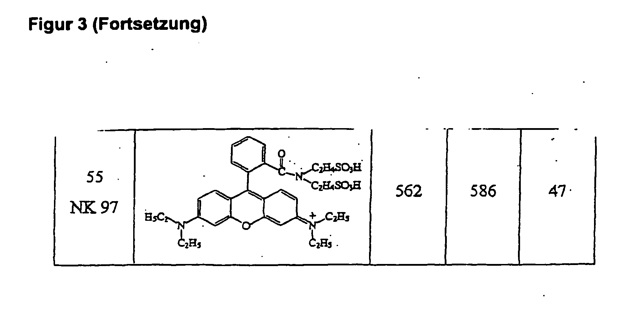

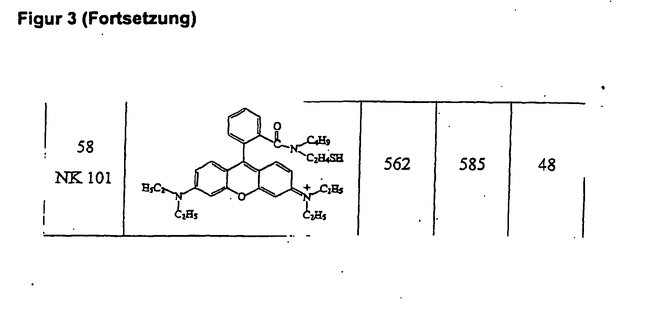

Die Wasserlöslichkeit eines Farbstoffs lässt sich beispielsweise verbessern, wenn R₅ oder/und R₆ etwa Sulfon- oder Phosphonsäuregruppen tragen (z.B. Fig. 3, Verb. 55) oder über Polyetherketten verfügen. Letztere machen die Verbindung auch in vielen organischen Lösungsmitteln besser löslich. Eine Art spezieller Polyether stellen etwa die für den fluoreszenzsensitiven Kationennachweis verwendeten Kronenether dar, die als Aza-Derivate auch über die Carboxamid-Methode an Farbstoffmoleküle gekoppelt werden können.

Weiterhin können beispielsweise Zuckerreste als R₅ oder/und R₆ eingeführt werden, wodurch eine hohe Wasserlöslichkeit erreicht werden kann.

In den erfindungsgemäßen Farbstoffen wird außerdem der Vorteil erreicht, dass durch unterschiedliche Bedeutungen von Y die Eigenschaften der Carboxamid-substituierten Farbstoffe je nach Verwendungszweck variiert werden können. So weisen beispielsweise Verbindungen mit Y = Selen die Eigenschaft auf, dass sie Fluoreszenz auslöschen. Solche Verbindungen sind u.a. wertvoll als Fluoreszenzquencher in der Bioanalytik.

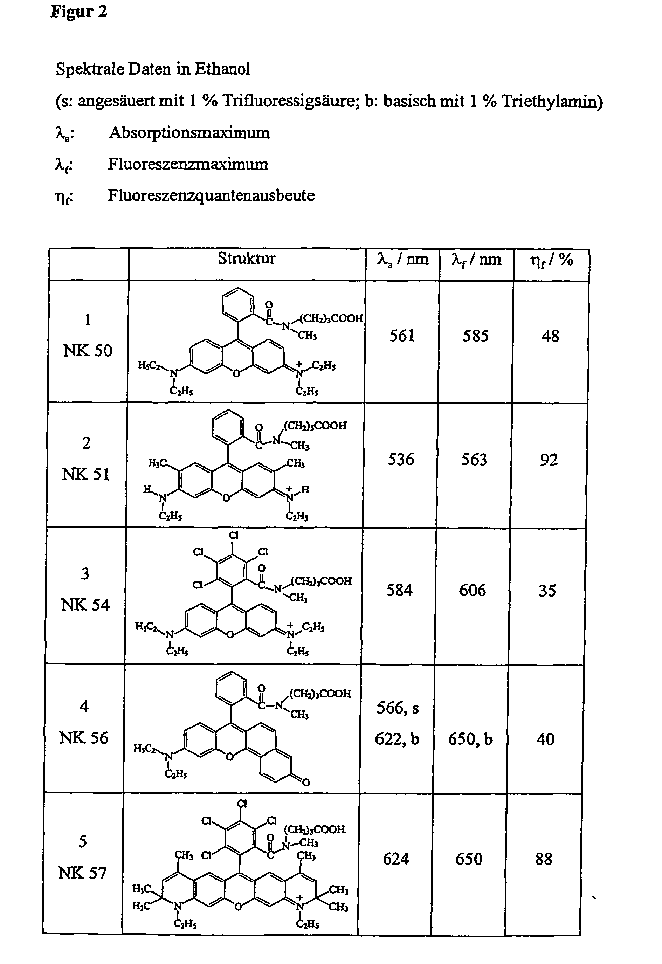

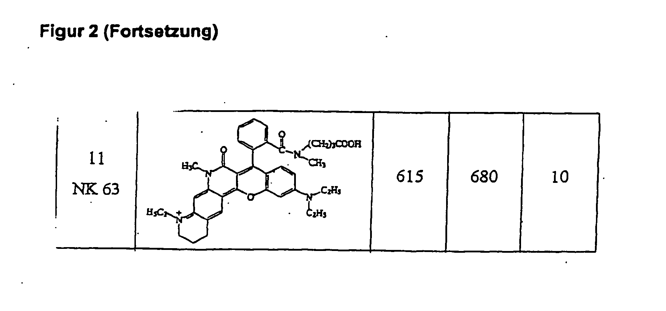

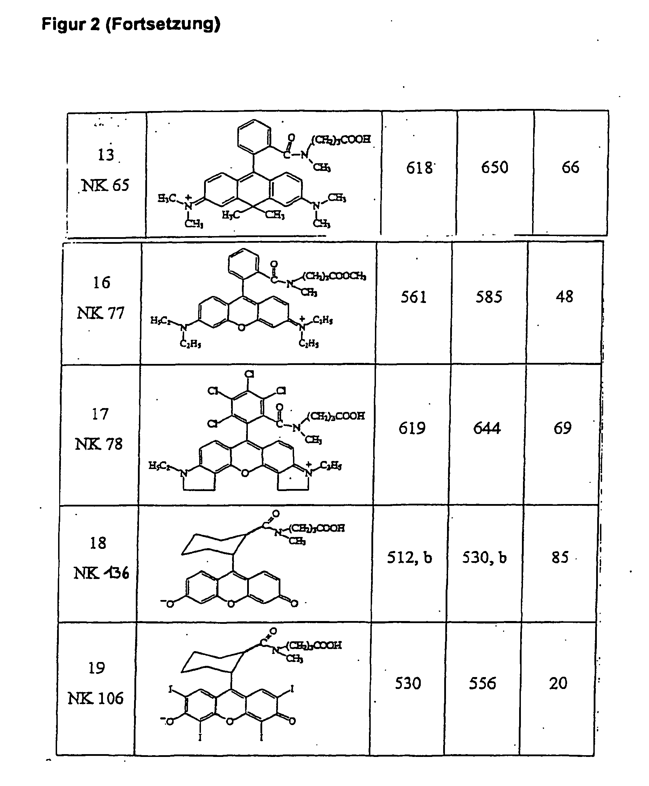

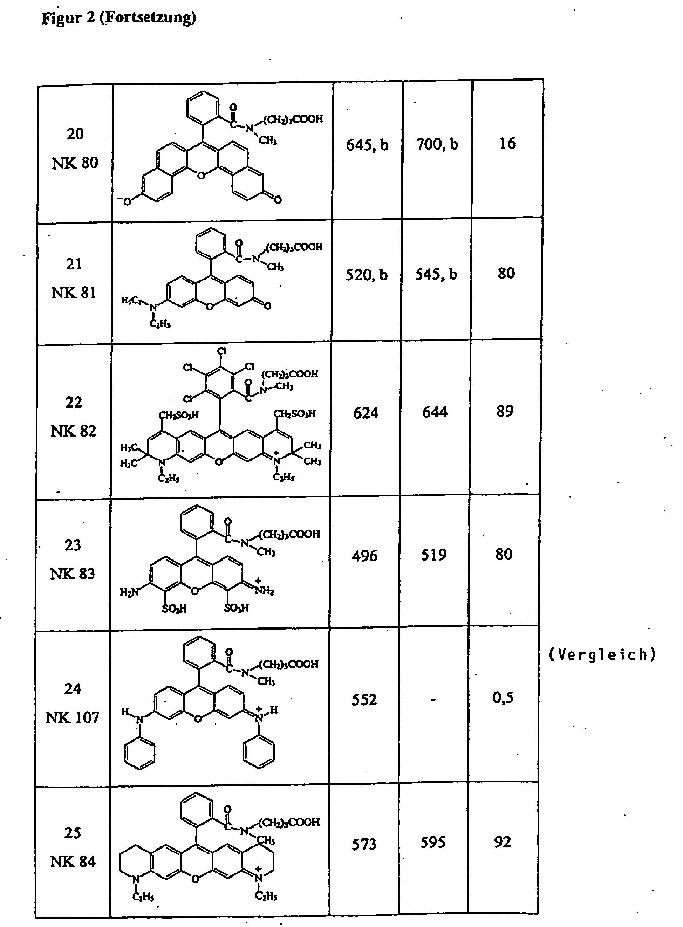

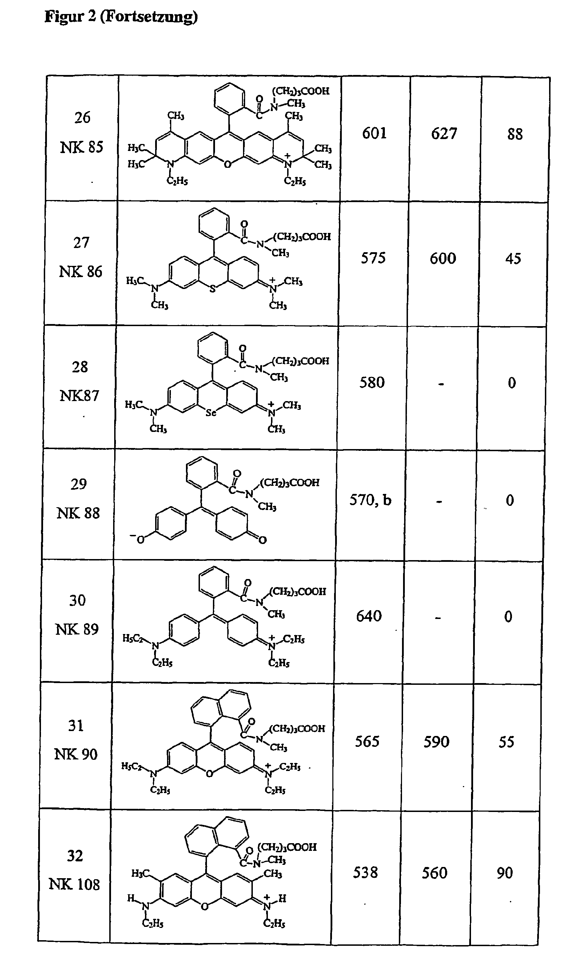

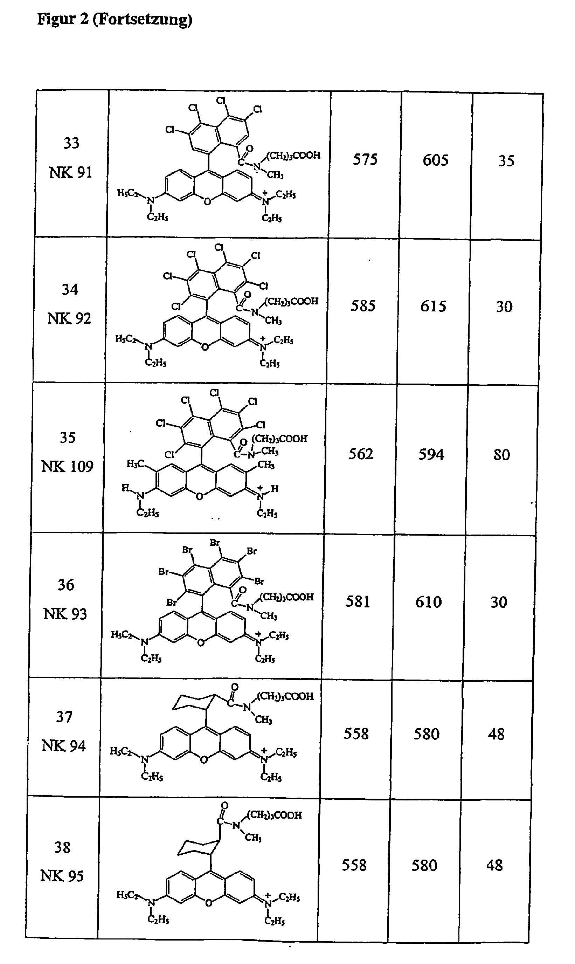

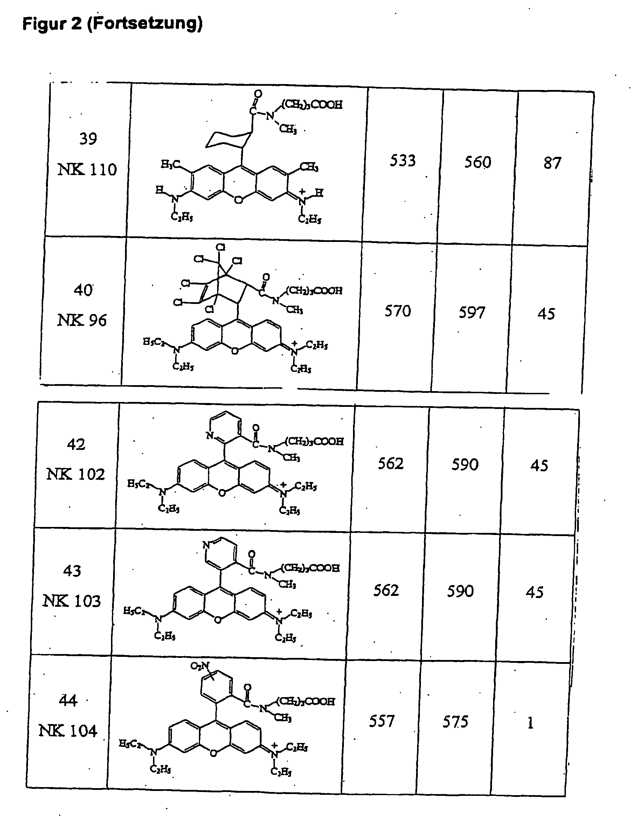

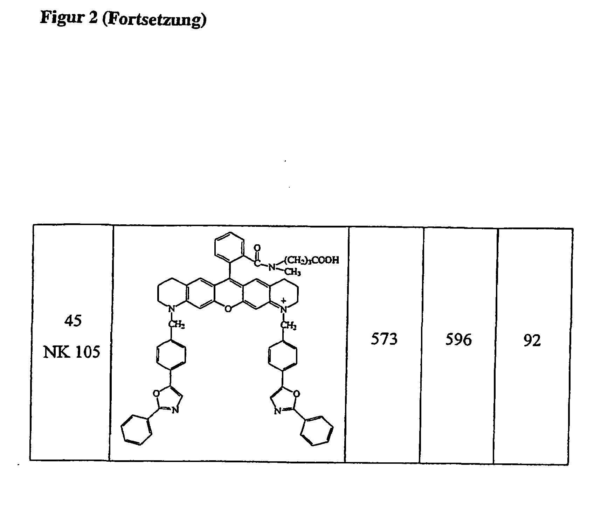

Verbindungen mit Y = CRₐR_{b} dagegen führen beispielsweise zu einer langwelligen Verschiebung in den roten Bereich verglichen mit Fluorescein bzw. entsprechenden Xanthen-Farbstoffen, wie Rhodaminen. Solche Verbindungen sind u.a. nützlich, um starkes Untergrundrauschen bei der Detektion, wie es herkömmlich verwendete Farbstoff-Derivate aufweisen, zu vermeiden. Ein Beispiel solcher erfindungsgemäßen Verbindungen ist die Verbindung 13 aus Figur 2 (NK65).

Farbstoffe, die der allgemeinen Formel (I) mit Y = -R₁₄ und -R₁₅ entsprechen, sind ebenfalls nützlich als Fluoreszenzquencher in der Analytik. Beispiele für solche Carboxamid-substituierten Triphenylmethan-Farbstoffe sind die erfindungsgemäßen Verbindungen 29 und 30 in Fig. 2 (NK88, NK 89).

Die Anmeldung offenbart weiterhin ein multichromophores System, in welchem ein Carboxamid-substituierter Farbstoff gemäß der allgemeinen Formel (I) über R₅ oder/und R₆ an ein oder mehrere weitere Farbstoffmoleküle gekoppelt ist.

Vorzugsweise stellt das multichromophore System ein System dar, in welchem ein weiteres Farbstoffmolekül gemäß der allgemeinen Formel (I) an einen erfindungsgemäßen Carboxamid-substituierten Farbstoff gekoppelt ist.

Es ist bevorzugt, dass die Kopplung an den oder die weiteren Carboxamid-substituierten Farbstoffe über jeweils den Rest R₅ oder/und R₆ erfolgt. Beispielsweise werden die Stickstoffatome der Carboxamidgruppe in den Farbstoffeinheiten über Alkylketten verknüpft.

Eine weitere bevorzugte Ausführungsform des multichromophoren Systems ist ein bichromophores System der Formel (III). 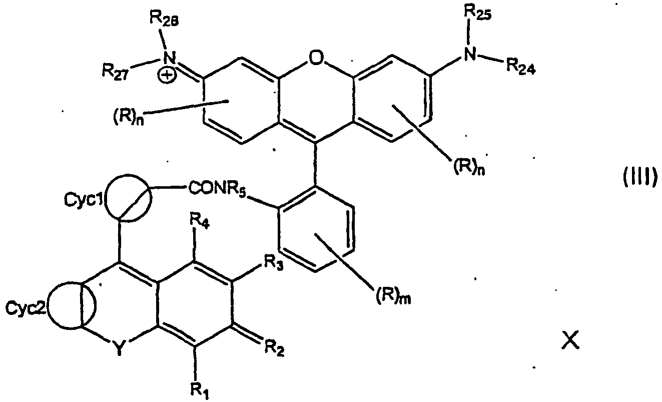 worin die Reste wie oben definiert sind und R₂₄, R₂₅ und R₂₆, R₂₇ wie R₇₁ R₈ definiert sind, wobei n unabhängig 0, 1, 2 oder 3 und m 0, 1, 2, 3 oder 4 bedeuten.

In den multichromophoren, insbesondere bichromophoren Systemen findet eine Energieübertragung (FRET) zwischen den Farbstoffmolekülen statt, wodurch bestimmte spektroskopische Eigenschaften erzielt werden können.

Die erfindungsgemäßen Carboxamid-substituierten Farbstoffe zeichnen sich insbesondere dadurch aus, dass sie die bei herkömmlichen Carboxylgruppen-substituierten Farbstoffen auftretende Lacton- bzw. Lactambildung nicht zeigen. Durch die Einführung der Carboxamid-Gruppe kann ferner erreicht werden, dass die Farbstoffmoleküle variabel funktionalisiert werden können, und somit für verschiedenste Anwendungen, wie analytische Verfahren, hervorragend geeignet sind. Überraschenderweise werden durch die Einführung der Carboxamid-Gruppe die sehr guten spektralen Eigenschaften der Ausgangsfarbstoffe, also die Absorptions- und Fluoreszenzbanden, die hohen Extinktionskoeffizienten sowie hohe Fluoreszenzquantenausbeuten und Stabilität nicht wesentlich verändert. Bei einigen Farbstofffen wurde lediglich beobachtet, dass das Absorptions- und Fluoreszenzmaximum der Farbstoffe um durchschnittlich 10 nm langwellig verschoben wird.

Des Weiteren wurde im Rahmen der vorliegenden Erfindung gefunden, dass ein Farbstoff mit einer entsprechenden zur Lacton- bzw. Lactambildung neigenden Carboxylgruppe (Formel (II)) durch einfache Umsetzung eines zuvor aus der Carboxylgruppe gebildeten aktivierten Derivats des Farbstoffs mit einem sekundären Amin in gängigen Lösungsmitteln, wie Acetonitril oder DMF, zu dem gewünschten erfindungsgemäßen Carboxamid-substituierten Farbstoff der allgemeinen Formel (I) reagiert. Dabei ist erstaunlich, dass die Amid-Bildung selbst bei variierenden sterischen und elektronischen Verhältnissen in dem Farbstoff-Vorläufer in guten Ausbeuten verläuft. Insbesondere war nicht zu erwarten, dass sich Carboxylgruppen-enthaltende Farbstoffe, welche sich hinsichtlich der elektronischen Verhältnisse in dem Farbstoffmolekül wesentlich von Fluorescein-Derivaten unterscheiden, dennoch erfolgreich zu den erfindungsgemäßen Carboxamid-substituierten Farbstoffen umwandeln lassen.

Ein weiterer Gegenstand der vorliegenden Erfindung ist daher ein Verfahren zur Herstellung von Carboxamid-substituierten Farbstoffen der allgemeinen Formel (I), umfassend die Schritte:
(a) Überführen der Carboxylgruppe eines Farbstoffs der allgemeinen Formel (II) 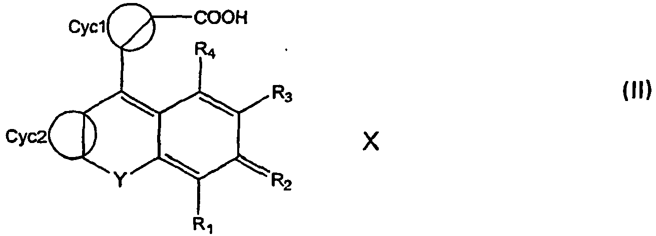 wobei die Reste wie oben angegeben definiert sind,
   in eine aktivierte Form;
(b) Umsetzen des in Schritt (a) erhaltenen aktivierten Farbstoffs mit einem sekundären Amin HNR₅R₆; und
(c) gegebenenfalls Isolieren des in Schritt (b) erhaltenen Carboxamid-substituierten Farbstoffs der allgemeinen Formel (I).

Die Aktivierung eines Carboxyl-enthaltenden Farbstoffs gemäß allgemeiner Formel (II) erfolgt vorzugsweise durch Überführen der Carboxyl-Gruppe in einen Aktivester oder ein Säurechlorid. Die Aktivierung erfolgt mit gängigen, dem Fachmann wohl bekannten Verfahren. Im Allgemeinen werden zur Herstellung der Aktivester gebräuchliche Reagenzien, wie z.B. N-Hydroxysuccinimid, N-Hydroxyphthalimid, N-Hydroxynaphthalimid, O-(N-Succinimidyl)-N,N,N',N'-Tetramethyluroniumtetrafluoroborat (TSTU) verwendet. Vorzugsweise wird bei Temperaturen von O °C bis etwa 60 °C, mehr bevorzugt bei 10°C bis 40 °C, am meisten bevorzugt bei 20°C bis 30 °C, insbesondere bei Raumtemperatur, gearbeitet.

Insbesondere wird bei der Herstellung von aktivierten von Rhodamin abgeleiteten Farbstoffen bei Raumtemperatur gearbeitet, um eine Zersetzung der Rhodamin-Derivate zu vermeiden. Die Reaktionszeiten variieren dabei je nach Struktur des Farbstoffes, im Allgemeinen ist die Reaktion jedoch nach etwa 5-48 Stunden, vorzugsweise 8-24 Stunden, abgeschlossen.

Die Umsetzung des in Schritt (a) erhaltenen aktivierten Farbstoffs erfolgt mit sekundären Aminen, welche die später im Carboxamid-substituierten Farbstoff der Formel (I) erwünschten Reste R₅ und R₆ enthalten. Die Umsetzung erfolgt vorzugsweise bei Raumtemperatur bis zu Temperaturen von ungefähr 100 °C, vorzugsweise bei Temperaturen von etwa 50 °C bis 90 °C, mehr bevorzugt ungefähr 60 °C bis 80°C. Dazu werden der Aktivester und das entsprechende sekundäre Amin in einem aprotischen Lösungsmittel, wie z.B. Acetonitril, DMF, DMSO etc., gelöst und erhitzt (Carboxamid-Verfahren). Insbesondere muss bei der Synthese der erfindungsgemäß bevorzugten Carboxamid-substituierten Farbstoffe, die von Rhodamin abgeleitet sind, darauf geachtet werden, dass das Lösungsmittel wasserfrei ist, um eine Reaktion mit dem Aktivester zu vermeiden.

Die Amid-Bildung ist im Allgemeinen nach mehreren Stunden vollständig abgelaufen. Gegebenenfalls wird der in Schritt (b) erhaltene Carboxamid-substituierte Farbstoff durch dem Fachmann wohl bekannte Verfahren, wie z.B. Extrahieren, Filtrieren, säulenchromatographische Aufarbeitung, Destillation etc., aus der Reaktionsmischung isoliert.

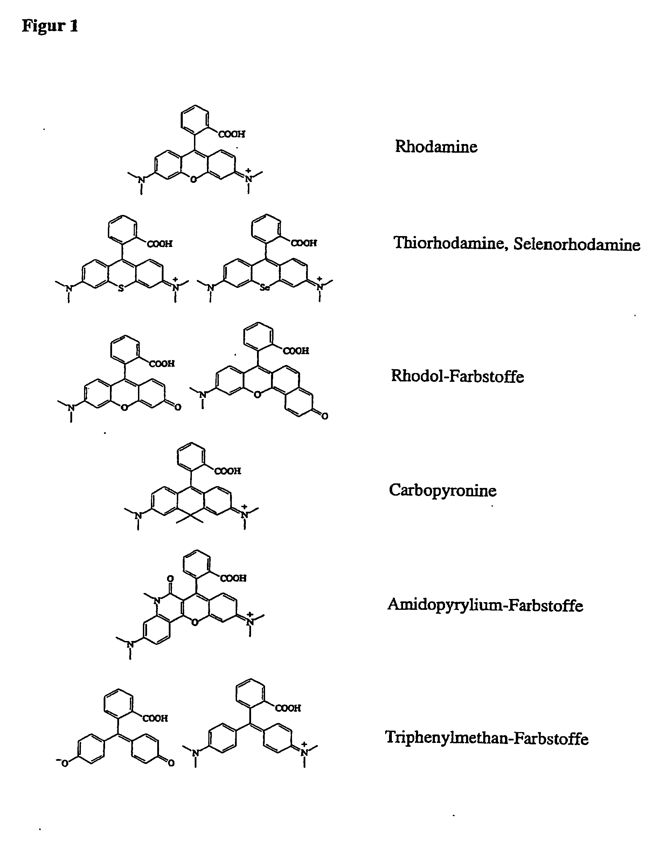

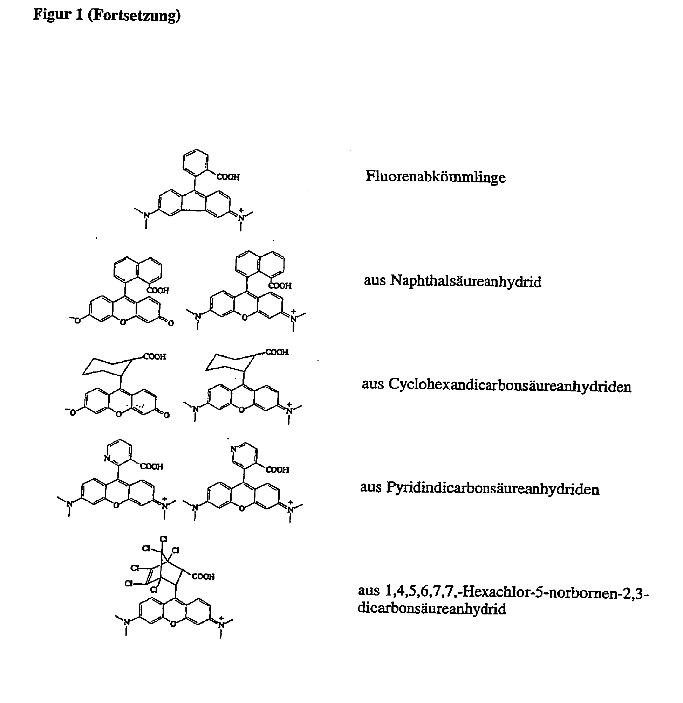

Ein wesentlicher Vorteil des beschriebenen Verfahrens ist die uneingeschränkte Anwendbarkeit auf alle Farbstoffe der allgemeinen Formel (II), welche insbesondere über eine ortho-ständige bzw. aufgrund ihrer Position zur Lactonbildung neigende Carboxyl-Gruppe verfügen. Beispiele für einsetzbare Farbstoffklassen sind in Figur 1 aufgeführt. Dabei ist vor allem bemerkenswert, dass mittels des erfindungsgemäßen Verfahrens auch Amino- und Imino- bzw. Iminiumgruppen-enthaltende Farbstoffen, wie z.B. Rhodamine, erfolgreich umgesetzt werden können.

Die entstehenden erfindungsgemäßen Farbstoffe sind besonders gut geeignet als Markierungsgruppen in analytischen Verfahren.

Ein weiterer Gegenstand der vorliegenden Erfindung ist daher die Verwendung eines erfindungsgemäßen Carboxamid-Farbstoffes gemäß der allgemeinen Formel (I) zur qualitativen oder/und quantitativen Bestimmung eines Analyten. Die erfindungsgemäßen Farbstoffe zeigen sowohl in chemischen als auch in medizinischen und biologischen Nachweisverfahren sehr gute Ergenbisse. Die Bestimmung kann beispielsweise in wässrigen Flüssigkeiten wie Körperflüssigkeiten, z.B. Blut, Serum, Plasma oder Urin, Abwasserproben oder Lebensmitteln mit dem Fachmann bekannten Nachweisverfahren durchgeführt werden. Dabei kann das Verfahren sowohl als Nasstest, z.B. in einer Küvette, oder als Trockentest auf einem entsprechenden Reagenzträger durchgeführt werden. Die Bestimmung der Analyten kann dabei über eine einzige Reaktion oder durch eine Sequenz von Reaktionen erfolgen.

Zum Nachweis eines Analyten wird der Carboxamid-substituierte Farbstoff der Erfindung vorzugsweise an den Analyten oder/und einen Träger gekoppelt. In einer besonders bevorzugten Ausführungsform der vorliegenden Erfindung wird der Farbstoff an einen Bestandteil eines Nachweisreagenzes gekoppelt. Ein solcher Bestandteil ist vorzugsweise ein universaler Rezeptor oder ein Analytanalogon. Beispiele für derartige Bindepartner sind bevorzugt ausgewählt aus Peptiden, Polypeptiden, Nukleinsäuren, Nukleosiden, Nukleotiden, Nukleinsäure-Analoga und Haptenen.

Der Nachweis umfasst insbesondere einen immunologischen Nachweis oder/und einen Nukleinsäure-Hybridisierungs-Nachweis. Der nachzuweisende Analyt ist vorzugsweise ausgewählt aus Peptiden, Polypeptiden, Antikörpern, Nukleinsäuren, Nukleinsäureanaloga, Haptenen, Zellen, Zellbestandteilen, Viren, viralen Bestandteilen, Metaboliten, Hormonen, Neurotransmittern und Arzneimitteln.

Als Träger kann jedes geeignete Material ausgewählt werden, z.B. poröses Glas, Kunststoffe, lonenaustauscher-Harze, Dextrane, Cellulose, Cellulosederivate und hydrophile Polymere.

Ein weiterer Gegenstand der vorliegenden Erfindung ist ein Konjugat eines Bindepartners mit einem Carboxamid-substituierten Farbstoff gemäß Formel (I). Der Bindepartner ist dabei vorzugsweise ausgewählt aus Peptiden, Polypeptiden, Nukleinsäuren, Nukleosiden, Nukleotiden, Nukleinsäure-Analoga und Haptenen. Die Konjugate können beispielsweise in Nukleinsäure-Hybridisierungsverfahren oder immunchemischen Verfahren verwendet werden. Derartige Verfahren sind beispielsweise beschrieben in Sambrook et al., Molecular Cloning, A Laboratory Manual, 1989, Cold Spring Harbor.

Die Kopplung an den nachzuweisenden Analyten oder/und den Bestandteil eines Nachweisreagenzes oder/und den Träger erfolgt vorzugsweise über die Substituenten R₅ oder/und R₆ des Carboxamid-substituierten Farbstoffes der allgemeinen Formel (I). Besonders bevorzugt bildet sich eine kovalente Bindung aus. Insbesondere wird dies mittels der dem Fachmann wohl bekannten Aktivestermethode bewerkstelligt. Insbesondere geeignet dafür sind Carboxyl-Gruppen oder Substituenten mit Carboxyl-Gruppen als Reste R₅ oder R₆. Eine solche Carboxyl-Gruppe ist ihrerseits wiederum aktivierbar und kann vorzugsweise für Kopplungen und Konjugatbildungen des erfindungsgemäßen Farbstoffs an Analyten, Bestandteile von Nachweisreagenzien oder Träger verwendet werden.

Beispielsweise setzt sich der aus dem Farbstoff NK 50 (Verbindung 1) hergestellte Succinimidylester (NHS-Ester) laut HPLC- bzw. DC-Analyse mit Benzylamin in Acetonitril innerhalb 30 Minuten vollständig zum entsprechenden Konjugat um. Auch mit Aminoethylmaleimid erhält man nach einigen Stunden eine vollständige Umsetzung zum Maleimid-Derivat. Diese Verbindung konnte wiederum erfolgreich mit N-Acetylcystein umgesetzt werden. Mit der beschriebenen Methode lassen sich aus der terminalen Carboxylgruppe alle gängigen Derivate herstellen, die z.B. zur Ankopplung an Amino- und Thiolgruppen von Biomolekülen verwendet werden.

Gegenüber herkömmlichen Farbstoffen weisen die erfindungsgemäßen Carboxamid-substituierten Farbstoffe den Vorteil auf, dass sie zwar über eine aktivierbare Carboxyl-Gruppe verfügen, diese jedoch nicht zur Lacton- bzw. Lactambildung neigt.

Durch die folgenden Figuren und Beispiele soll die Erfindung näher erläutert werden.

### Figuren

**In** **Figur 1** sind Vertreter verschiedener Carboxyl-Gruppen enthaltender Farbstoffklassen aufgeführt, wobei die Substituenten weggelassen bzw. nicht näher bezeichnet sind.
**In** **Figur 2** sind erfindungsgemäße Carboxamid-substituierte Farbstoffe mit koppelbarer Carboxyl-Gruppe an R₅ bzw. R₆ mit den jeweiligen spektralen Daten (Absorptionsmaximum, Fluoreszenzmaximum und Fluoreszenzquantenausbeute) aufgeführt.
In **Figur 3** sind weitere Beispiele für erfindungsgemäße Carboxamid-substituierte Farbstoffe und deren spektrale Daten (Absorptionsmaximum, Fluoreszenzmaximum und Fluoreszenzquantenausbeute) angegeben.

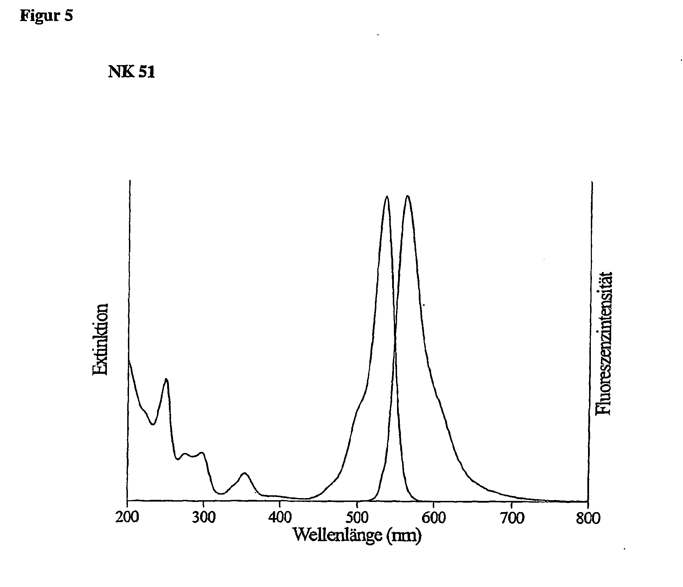

**Figur 5** zeigt das Absorptions- bzw. Fluoreszenzspektrum von Verbindung NK51 in Ethanol.

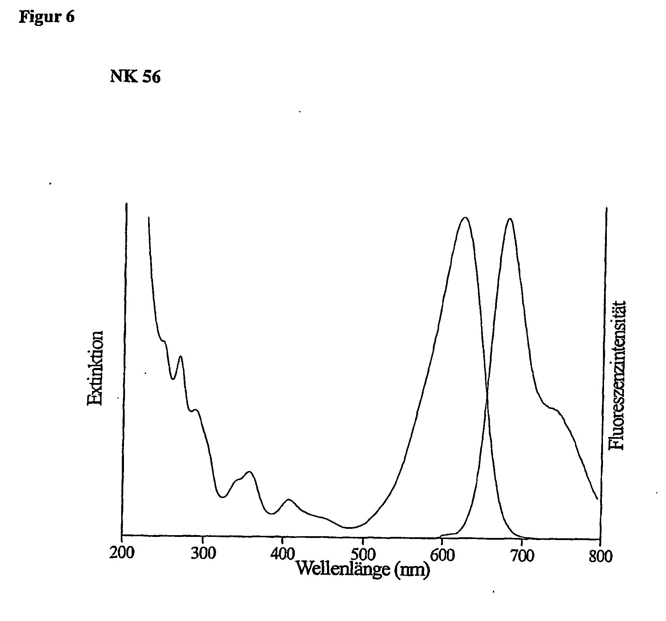

**Figur 6** zeigt das Absorptions- bzw. Fluoreszenzspektrum von Verbindung NK56 in Ethanol.

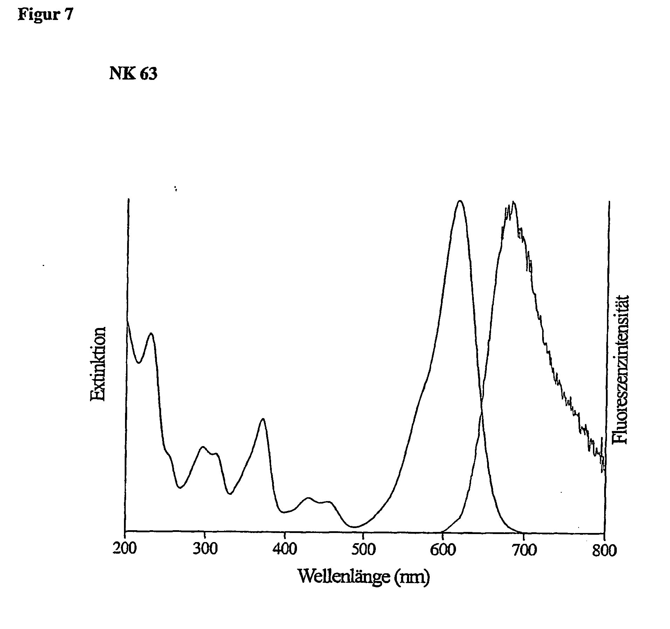

**Figur 7** zeigt das Absorptions- bzw. Fluoreszenzspektrum von Verbindung NK63 in Ethanol.

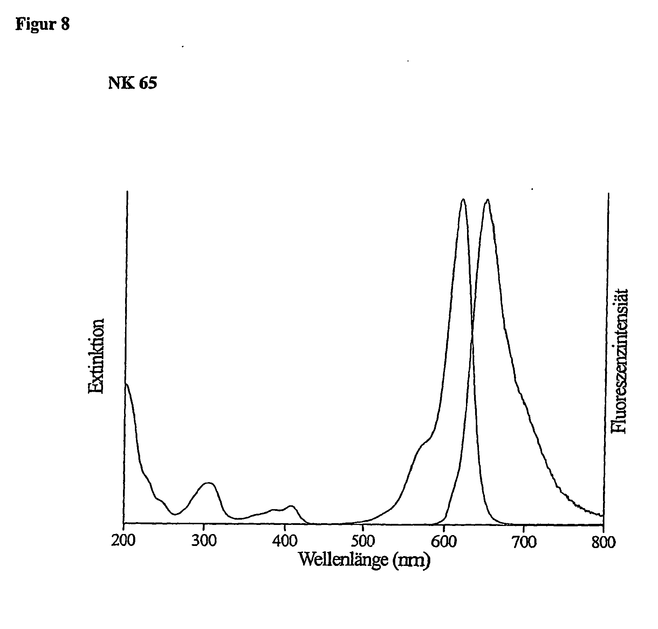

**Figur 8** zeigt das Absorptions- bzw. Fluoreszenzspektrum von Verbindung NK65 in Ethanol.

### Beispiele

Die für das Carboxamidverfahren verwendeten Farbstoffe sind entweder kommerziell erhältlich oder nach literaturbekannten Synthesen oder dem Fachmann bekannten Verfahren darzustellen. Die verwendeten sekundären Amine sind ebenfalls in den meisten Fällen kommerziell erhältlich oder nach literaturbekannten Methoden zugänglich. Das bevorzugte Verfahren zur Herstellung erfindungsgemäßer Verbindungen ist exemplarisch anhand der Strukturen 1 (NK 50), 4 (NK 56) und 18 (NK36 beschrieben.

### Beispiel 1: Herstellung erfindungsgemäßer Verbindungen

### Verbindung 1 (NK 50)

### 1. Stufe: Rhodamin B-NHS-Ester

1 g (2,1 mmol) käufliches Rhodamin B (Chlorid) und 700 mg (2,4 mmol) O-(N-Succinimidyl)-N,N,N',N'-tetramethyluroniumtetrafluoroborat (TSTU) werden zusammen mit 700µl Hünigbase (N-Ethyl-diisopropylamin) in 50 ml trockenem Acetonitril aufgenommen und bei Raumtemperatur für etwa zwei Stunden verrührt und die Reaktion dünnschichtchromatographisch kontrolliert. Nach beendeter Umsetzung wird von wenig farblosem Niederschlag abfiltriert und das Filtrat mit 2 ml Perchlorsäure (60 %ig) versetzt. Durch zügiges Zutropfen von eiskalter 10 %iger Natriumperchloratlösung wird der Aktivester ausgefällt, abgesaugt, mit Eiswasser und Diethylether gewaschen und schließlich im Vakuumexsikkator über Phosphorpentoxid scharf getrocknet.
Ausbeute: 0,8 g
ESI-Massenspektrum: m/z = 540,2

### 2. Stufe: NK 50

0,5 g (0,78 mmol) Rhodamin B-NHS-Ester (Perchlorat) und 0,25 g (1,56 mmol) 4-Methylaminobuttersäure-hydrochlorid werden in 40 ml Acetonitril suspendiert, mit 270 µl Hünigbase versetzt und zum Rückfluss erhitzt. Die Reaktion wird dünnschichtchromatographisch kontrolliert und nach dem Verschwinden des NHS-Esters - in der Regel vier Stunden - abgebrochen. Die Reaktionsmischung wird zur Trockene eingeengt und an neutralem Aluminiumoxid (Aktivität I) säulenchromatographisch gereinigt. Der Gradient des Elutionsmittels läuft von Ethanol zu Wasser. Die Farbstofffraktionen werden gesammelt und einrotiert. Der Rückstand wird zur Entfernung von 4-Methylaminobuttersäure-Resten in Chloroform gelöst und gegen mit Natriumchlorid gesättigtes Wasser ausgeschüttelt. Die Chloroformphase wird getrocknet und einrotiert.
Ausbeute: 0,3 g
¹H-NMR-Daten in DMSO-d₆:
δ 1,2 (T, 12H, -CH₃); 1,6 (M, 2H, -CH₂-); 2,6 (D, 2H, -CH₂-); 2,8 (S, 3H, -CH₃); 3,1 (D, 2H, -CH₂-); 3,6 (Qu, 8H, N-CH₂-); 6,9 (S, 2H, ArH); 7,1 (M, 4H, ArH); 7,6 (M, 4H, ArH)
ESI-Massenspektrum: m/z = 542,2

Anhand des Farbstoffes NK 50 (Verbindung 1) konnte gezeigt werden, dass die gebildete sekundäre Amidbindung unter den gängigen physiologischen bzw. experimentellen Bedingungen stabil ist. Die Carboxamidbindung wird bei Raumtemperatur innerhalb von 24 Stunden und bei pH-Werten zwischen pH 3 und pH 11 nicht gespalten. Bei erhöhter Temperatur (100 °C) findet eine merkliche Spaltung erst im pH-Bereich oberhalb pH 11 statt.

### Verbindung 4 (NK 56)

### 1. Stufe: MR 33-NHS-Ester

300 mg (0,69 mmol) MR 33 werden mit 157 mg (0,75 mmol) Dicyclohexylcarbodiimid und 87 mg (0,75 mmol) N-Hydroxy-succinimid in 15 ml Acetonitril gelöst und bei Raumtemperatur für 24 Stunden verrührt. Die Reaktionsmischung wird filtriert und das Filtrat anschließend mit Diethylether versetzt. Der dabei entstehende Niederschlag wird abgesaugt und getrocknet.
Ausbeute: 80 mg

### 2. Stufe: NK 56

40 mg (0,075 mmol) MR 33-NHS-Ester werden zusammen mit 25 mg (0,15 mmol) 4-Methylaminobuttersäure-hydrochlorid und 30 µl Hünigbase in 20 ml Acetonitril gelöst und für etwa eine Stunde zum Rückfluss erhitzt. Die Reaktion wird dünnschichtchromatographisch kontrolliert. Nach Beendigung der Umsetzung wird zur Trockne einrotiert und der Rückstand an Kieselgel mit einem von Chloroform über Ethanol zu Wasser verlaufenden Gradienten säulenchromatgraphisch gereinigt.

### Verbindung 51 (NK 67) (nicht erfindungsgemäß)

0,5 g (0,78 mmol) Rhodamin B-NHS-Ester (Perchlorat) und 0,3 g (1,56 mmol) N-Methyl-D-glucamin werden in 20 ml Acetonitril gelöst und zum Rückfluss erhitzt. Die Isolierung des Produktes erfolgt säulenchromatographisch an Kieselgel mit Chloroform/Ethanol 9:1.
ESI-Massenspektrum: m/z = 620,3

### Verbindung 18 (NK 136)

### 1. Stufe: NK 135

11 g Resorcin (100 mmol) und 10,8 g Cyclohexandicarbonsäureanhydrid (isomerengemisch, 70 mmol) werden gemischt, fein gemörsert und auf dem Ölbad bei etwa 180 °C aufgeschmolzen. Im Verlauf der Reaktion wird die Schmelze fest. Die Reaktion ist nach ca. 4 Stunden beendet. Nach dem Erkalten wird der Feststoff zermörsert und mit Wasser ausgekocht, abgesaugt und gründlich mit Wasser gewaschen.

Das rohe Produkt kann für die nächste Stufe ohne Reinigung eingesetzt werden. Zur weiteren Reinigung kann das Produkt in verdünnter Natronlauge aufgelöst und mit halbkonzentrierter Schwefelsäure wieder ausgefällt werden.

### 2. Stufe: NK 135-NHS-Ester

100 mg (0,3 mmol) NK 135 werden in 10 ml Acetonitril unter Zusatz von etwa 100 µl DMF und 0,5 ml 20 %iger wässriger Tetraethylammoniumhydroxidlösung gelöst und mit 72 mg (0,35 mmol) Dicyclohexylcarbodiimid und 40 mg (0,35 mmol) N-Hydroxysuccinimid versetzt. Die Lösung wird für 12 Stunden bei Raumtemperatur verrührt, dann im Vakuum bei etwa 40 °C zur Hälfte eingeengt, filtriert und anschließend wird durch Zugabe von Wasser ein Feststoff ausgefällt. Das Rohprodukt wird abgesaugt, mit wenig Wasser gewaschen und im Vakuum über Phosphorpentoxid getrocknet. Der Feststoff enthält auch nicht umgesetztes Edukt.

### 3. Stufe: NK 136

100 mg des so erhaltenen Feststoffs werden mit 200 mg 4-Methylaminobuttersäure-hydrochlorid in 10 ml Acetonitril suspendiert und mit 250 µl Hünigbase für drei Stunden zum Rückfluss erhitzt. Die Reaktionslösung wird zur Trockne einrotiert und an Kieselgel mit einem von Chloroform zu Ethanol verlaufenden Gradienten säulenchromatographisch isoliert. Die letzte farbige Zone enthält das gewünschte Produkt.
ESI-Massenspektrum: m/z = 434,1

### Beispiel 2: Beispiele zur Konjugatbildung

### NK 50-NHS-Ester

0,5 g (0,78 mmol) NK 50-Perchlorat werden in 20 ml trockenem Acetonitil gelöst, mit 250 mg (0,85 mmol) TSTU und 170 µl (1 mmol) Hünigbase versetzt und bei Raumtemperatur gerührt. Nach beendeter Umsetzung wird die Lösung im Vakuum auf etwa ein Viertel eingeengt, mit 1 ml 60 %iger Perchlorsäure versetzt und durch Zutropfen von 20 %iger Natriumperchloratlösung der Farbstoff ausgefällt. Der Feststoff wird abgesaugt, gründlich mit Wasser und Diethylether gewaschen und im Vakuum über Phosphorpentoxid im Ölpumpenvakuum getrocknet.
Ausbeute: 0,4 g
ESI-Massenspektrum: m/z = 639,3

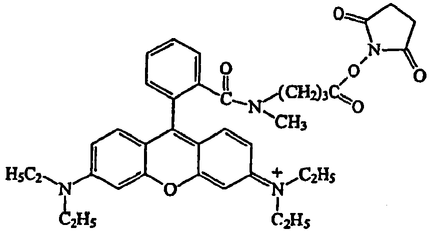

### NK 50-Aminoethyl-maleinimid

0,1 mmol NK 50-NHS-Ester werden in 5 ml trockenem Acetonitril gelöst, mit 0,15 mmol Aminoethyl-maleinimid und 30 µl Hünigbase versetzt. Die Lösung wird für 5 Stunden bei Raumtemperatur verrührt, filtriert und in Diethylether getropft. Der so erhaltene Niederschlag wird im Ölpumpenvakuum getrocknet.

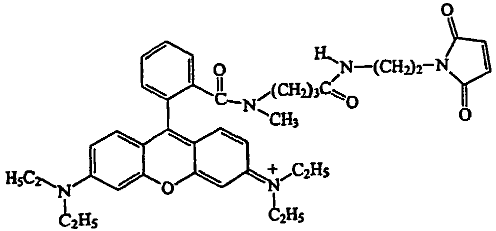

### NK 50-Aminoethyl-maleinimid-Cystein-Konjugat

0,1 mmol NK 50-Aminoethyl-maleinimid werden in 20 ml Ethanol gelöst und portionsweise mit insgesamt 0,15 mmol Cystein versetzt. Man verrührt bei Raumtemperatur und tropft nach etwa 2 Stunden ca. 50 ml einer 10 %igen Natriumperchloratlösung hinzu. Der ausgefallene Feststoff wird abfiltriert und im Vakuumexsikkator über Phosphorpentoxid getrocknet.

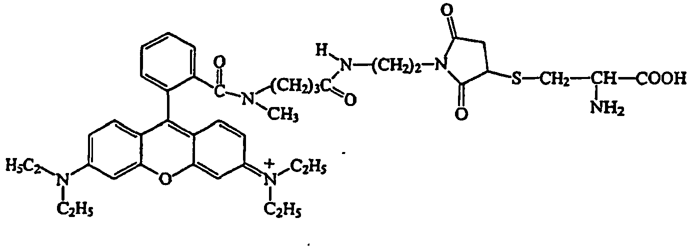

### NK 50-dUTP-Konjugat

10 µmol 5-(3-Aminoallyl)-dUTP werden in 0,5 ml 0,1 M Natriumborat-Puffer (pH 8) gelöst und mit einer Lösung von 5 µmol NK 50-Aktivester in 1 ml aminfreiem N,N-Dimethylformamid versetzt. Die Lösung wird 15 Stunden bei Raumtemperatur gerührt, das Lösungsmittel im Vakuum abdestilliert und der Rückstand chromatographisch gereinigt.

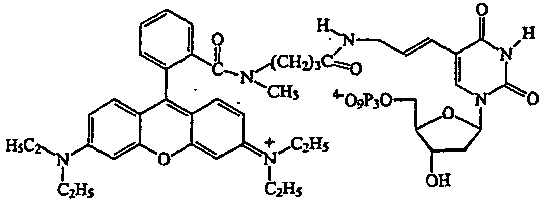

### NK 50-Digoxin-3-Carboxymethylether-Diaminodioxaoctan-Konjugat (Dig-CME-DADOO)

0,02 mmol NK 50-Aktivester werden mit 0,02 mmol Dig-CME-DADOO in Acetonitril für 18 Stunden bei Raumtemperatur verrührt. Die Lösung wird eingeengt und der Rückstand chromatographisch gereinigt.

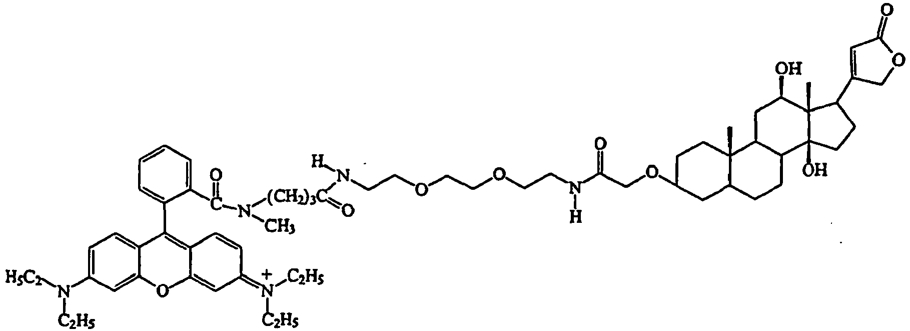

## Patentansprüche

1. Carboxamid-substituierter Farbstoff der allgemeinen Formel (I) 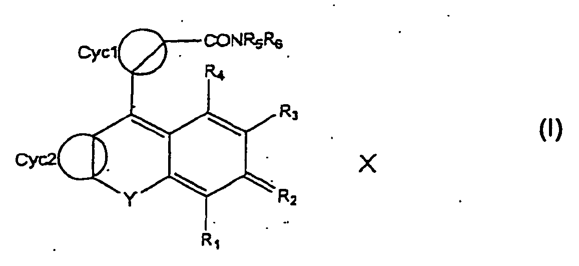 wobei
Y = Sauerstoff, Schwefel, Selen, CRₐR_{b}, NR_{c}, eine direkte Verknüpfung oder -R¹⁴ und -R¹⁵ darstellt;
R₁, R₃, R₄ unabhängig Wasserstoff, Halogen, -O^{e}, eine Hydroxygruppe; Thiolgruppe, Aminogruppe, Ammoniumgruppe, Sulfogruppe, Phosphogruppe, Nitrogruppe, Carbonylgruppe, Carboxygruppe, ein Carbonsäurederivat, eine Nitrilgruppe, Isonitrilgruppe, Cyanatgruppe, Isocyanatgruppe, Thiocyanatgruppe, Isothiocyanatgruppe oder eine geradkettige, verzweigte oder cyclische gesättigte oder ungesättigte Kohlenwasserstoffgruppe mit bis zu 40 Kohlenstoffatomen darstellen;
R_{a,} R_{b,} R_{c} und R_{14,} R₁₅ unabhängig die gleichen Bedeutungen haben, wie es für R_{1,} R₃, R₄ definiert ist;
R₂ = O; oder 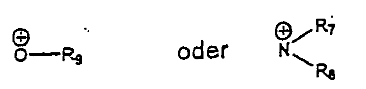
darstellt, wobei
R_{7,} R₈, R₉ unabhängig Wasserstoff oder eine geradkettige, verzweigte oder cyclische gesättigte Kohlenwasserstoffgruppe mit bis zu 40 Kohlenstoffatomen darstellen, welche Heteroatome enthalten kann und unsubstituiert oder substituiert sein kann oder
R₁ zusammen mit **R₂** 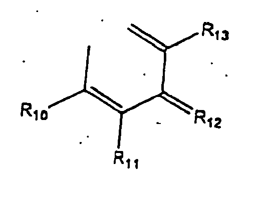
darstellt, wobei
R₁₀, R₁₁, R₁₃ die gleichen Bedeutungen haben, wie es für R_{1,} R₃, R₄ definiert ist;
R₁₂ = O, oder 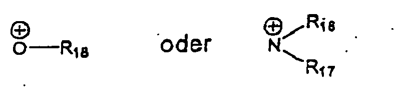
darstellt, wobei
R₁₆, R_{17'} R₁₈ die gleichen Bedeutungen aufweisen, wie es für R₇, R₈, R₉ definiert ist;
R₅ eine geradkettige, verzweigte oder cyclische gesättigte oder ungesättigte Kohlenwasserstoffgruppe mit bis zu 40 Kohlenstoffatomen darstellt, welche Heteroatome enthalten kann und unsubstituiert oder substituiert sein kann;
R₆ eine geradkettige oder verzweigte gesättigte oder ungesättigte Kohlenwasserstoffgruppe mit bis zu 40 Kohlenstoffatomen darstellt, welche Heteroatome enthalten kann und einen Substituenten ausgewählt aus einer Carboxylgruppe, Cyanatgruppe, iminogruppe, Iminiumgruppe, Isocyanatgruppe, Isothiocyanatgruppe, Nitrogruppe, Phosphogruppe, . Sulfogruppe, Thiocyanatgruppe, Thioethergruppe, Thiolgruppe oder einem Carbonsäurederivat umfasst;
Cyc1 einen organischen Rest darstellt, der ein Ringsystem umfasst, ausgewählt aus aromatischen, heteroaromatischen, chinoiden und cycloaliphatischen Ringen;
Cyc2 einen organischen Rest darstellt, der ein Ringsystem umfasst, ausgewählt aus aromatischen, heteroaromatischen, chinoiden und cycloaliphatischen Ringen;
wobei jeder der Reste in dem Farbstoff der formel (I) mit einem oder mehreren benachbarten Resten ein Ringsystem bilden kann;
und X, wenn es zum Ladungsausgleich erforderlich ist, ein oder mehrere ein- oder mehrwertige Anionen darstellt;
mit der Maßgabe, dass in der allgemeinen Formel (I) nicht gleichzeitig
- Y = Sauerstoff,
- Cyc1 = Phenyl oder substituiertes Phenyl,
- Cyc2 = Hydroxy-, Ether- oder Ester-substituiertes Phenyl
und
- R₂=O
darstellen.

2. Carboxamid-substituierter Farbstoff nach Anspruch 1, wobei Cyc2 einen Stickstoff-enthaltenden Heterocyclus oder ein Ringsystem darstellt, welches mit mindestens einer Aminogruppe substituiert ist
oder/und R₂ = 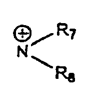 darstellt oder
zusammen mit R₁= 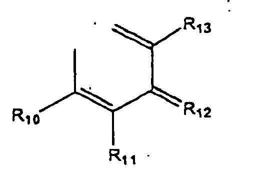 darstellt,
wobei R₁₂ = 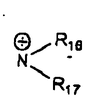 ist, wobei R₇, R₈; R₁₀, R_{11,} R₁₃ und R₁₆, R₁₇ wie in Anspruch 1 definiert sind.

3. Carboxamid-substituierter Farbstoff nach Anspruch 1 oder 2, wobei Cyc2 in Formel (I) eine Struktur (A), (B), (C), (D), (E), (F), (G), (H) oder (J) aufweist, 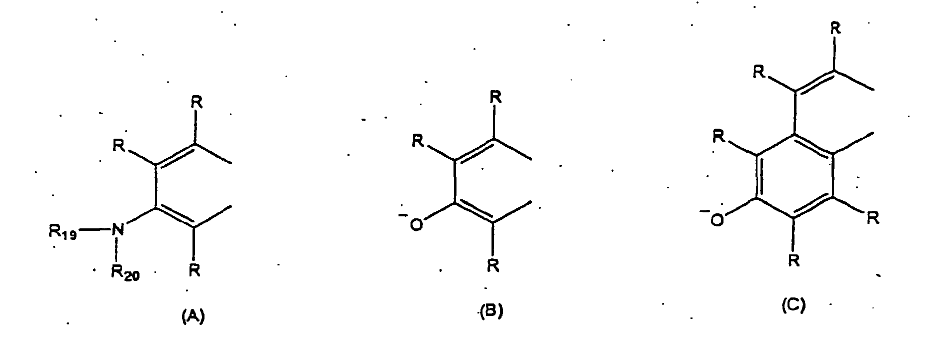 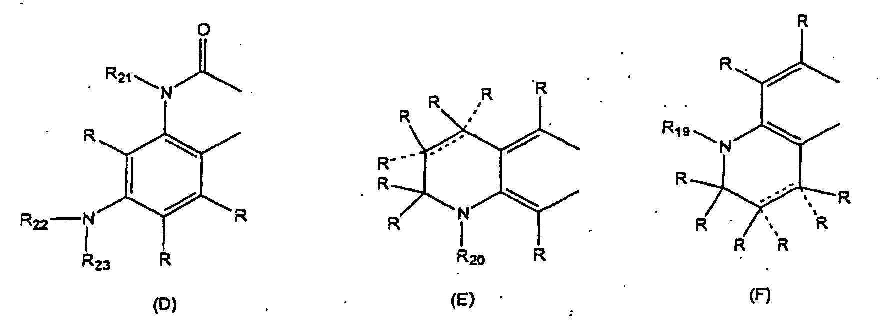 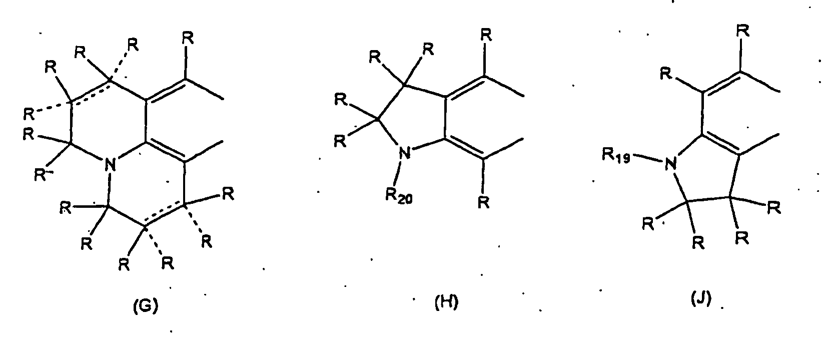
wobei R jeweils unabhängig wie R₁, R₃, R₄ in Anspruch 1 definiert ist;
R₁₉, R₂₀ und R₂₂, R₂₃ unabhängig wie R₇, R₆ in Anspruch 1 definiert sind; und
R₂₁ wie R₇ in Anspruch 1 definiert ist und die gestrichelten Linien gegebenenfalls Doppelbindungen bedeuten, bei deren Vorhandensein die über eine gestrichelte Linie gebundenen Reste fehlen.

4. Carboxamid-substituierter Farbstoff nach einem der vorhergehenden Ansprüche, wobei Cyc1 substituiertes oder unsubstituiertes Phenyl, Naphthyl, Pyridyl oder Cyclohexyl darstellt.

5. Carboxamid-substituierter Farbstoff nach einem der vorhergehenden Ansprüche, wobei
R₂= 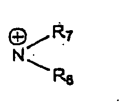 darstellt, worin R₇ und R₈ wie in Anspruch 1 definiert sind.

6. Carboxamid-substituierter Farbstoff nach Anspruch 5, wobei R₁ mit R₈ oder/und R₃ mit R₇ verbrückt ist und ein Ringsystem bildet.

7. Carboxamid-substituierter Farbstoff nach Anspruch 6, wobei das oder die Ringsystem/e 5- oder 6-gliedrige Ringe enthalten.

8. Carboxamid-substituierter Farbstoff nach Anspruch 7, wobei ein Ringsystem der Struktur (K), (L), (M), (N) oder (O) gebildet wird: 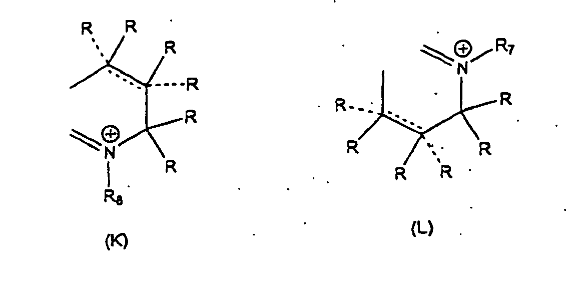 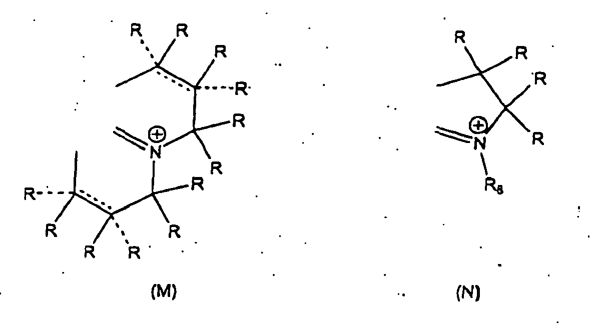 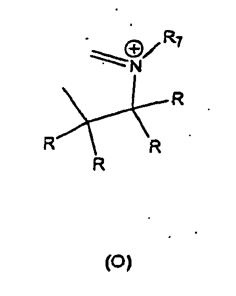 wobei R jeweils unabhängig wie R₁, R₃, R₄ und R₇, R₈ wie in Anspruch 1 definiert sind,
und die gestrichelten Linien gegebenenfalls Doppelbindungen bedeuten, bei deren Vorhandensein die über eine gestrichelte Linie gebundenen Reste fehlen.

9. Carboxamid-substituierter Farbstoff nach einem der Ansprüche 1 bis 4, wobei
R₂ zusammen mit R₁ 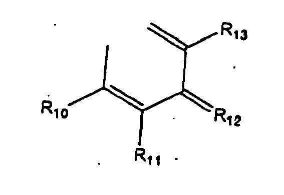 darstellt, worin R₁₀ - R₁₃ wie in Anspruch 1 definiert sind.

10. Carboxamid-substituierter Farbstoff nach Anspruch 9, wobei
R₁₂ = O darstellt.

11. Carboxamid-substituierter Farbstoff nach Anspruch 9, wobei R₁₂ = 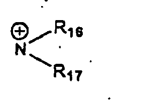 darstellt, worin R₁₆ und R₁₇ wie in Anspruch 1 definiert sind.

12. Carboxamid-substituierter Farbstoff nach einem der vorhergehenden Ansprüche, wobei Y = Sauerstoff darstellt.

13. Carboxamid-substituierter Farbstoff nach einem der Ansprüche 1 bis 11, wobei Y = Schwefel, Selen oder CRₐR_{b} darstellt, wobei Rₐ und R_{b} wie in Anspruch 1 definiert sind.

14. Carboxamid-substituierter Farbstoff nach einem der Ansprüche 1 bis 11, wobei Y = die Reste -R₁₄ und -R₁₅ darstellt, wobei R₁₄ und R₁₅ wie in Anspruch 1 definiert sind.

15. Carboxamid-substituierter Farbstoff nach Anspruch 8, wobei Cyc1 gegebenenfalls substituiertes Phenyl ist, Cyc2 die Struktur (E) aufweist und Y = Sauerstoff ist und R₇ und R₃ ein Ringsystem (K) bilden, R₇ und R₃ wie in Anspruch 1 definiert sind.

16. Carboxamid-substituierter Farbstoff nach Anspruch 8, wobei Cyc1 gegebenenfalls substituiertes Phenyl ist, Cyc2 die Struktur (A) aufweist und Y = Schwefel, Selen oder CRₐR_{b} darstellt, wobei Rₐ und R_{b} wie in Anspruch 1 definiert sind.

17. Verfahren zur Herstellung von Carboxamid-substituierten Farbstoffen der allgemeine Formel (I) gemäß einem der Ansprüche 1 bis 16, umfassend die Schritte:
(a) Überführen der Carboxylgruppe eines Farbstoffs der allgemeine Formel (II) 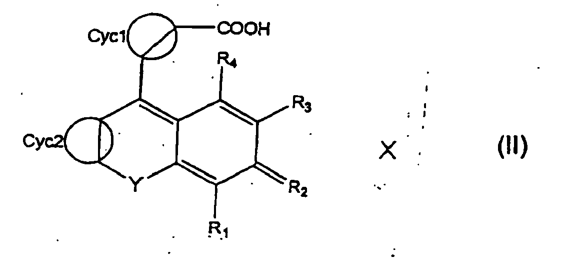 wobei die Reste wie in Anspruch 1 angegeben definiert sind,
in eine aktivierte Form;
(b) Umsetzen des in Schritt (a) erhaltenen aktivierten Farbstoffs mit einem sekundären Amin HNR₅R₆; und
(c) gegebenenfalls Isolieren des in Schritt (b) erhaltenen Carboxamid-substituierten Farbstoffs der allgemeinen Formel **(I).**

18. Verfahren nach Anspruch 17, wobei Schritt (a) bei Temperaturen von Raumtemperatur bis 60°C durchgeführt wird.

19. Verfahren nach Anspruch 17 oder 18, wobei in Schritt (b) ein aprotisches Lösungsmittel verwendet wird.

20. Verfahren nach einem der Ansprüche 17 bis 19, wobei zur Aktivierung N-Hydroxysuccinimid, N-Hydroxyphthalimid, N-Hydroxynaphthalimid, O-(N-Succinimidyl)-N,N,N',N'-Tetramethyl-uronimtetrafluoroborat (TSTU) verwendet wird.

21. Verwendung eines Carboxamid-Farbstoffes gemäß einem der Ansprüche 1 bis 16 zur qualitativen oder/und quantitativen Bestimmung eines Analyten.

22. Verwendung nach Anspruch 21, wobei der Carboxamid-substituierte Farbstoff der allgemeinen Formel **(I)** an den nachzuweisenden Analyten oder/und an einen Bestandteil eines Nachweisreagenzes oder/und einen Träger gekoppelt wird.

23. Verwendung nach Anspruch 21 oder 22, wobei der Nachweis einen immunologischen Nachweis oder/und einen Nukleinsäure-Hybridisierungs-Nachweis umfasst.

24. Konjugat aus einem Carboxamid-substituierten Farbstoff der allgemeinen Formel (I) nach einem der Ansprüche 1 bis 16 und einem Bindepartner.

25. Konjugat nach Anspruch 24, wobei der Bindepartner ausgewählt ist aus Peptiden, Polypeptiden, Nukleinsäuren, Nukleosiden, Nukleotiden, Nukleinsäure-Analoga und Haptenen.

26. Verwendung eines Konjugats gemäß Anspruch 24 oder 25 in Nukleinsäure-Hybridisierungsverfahren und immunochemischen Verfahren.

27. Verwendung nach Anspruch 22 oder 23, wobei die Kopplung an den nachzuweisenden Analyten oder/und den Bestandteil eines Nachweisreagenz oder/und den Träger über die Substituenten R₅ oder/und R₆ des Carboxamid-substituierten Farbstoffs der allgemeinen Formel (I) erfolgt, wobei die Reste R₅ und R₆ wie in Anspruch 1 definiert sind.

28. Verwendung nach Anspruch 27, wobei die Kopplung über eine kovalente Bindung erfolgt.

## Claims

1. Carboxamide-substituted dye of the formula (I) 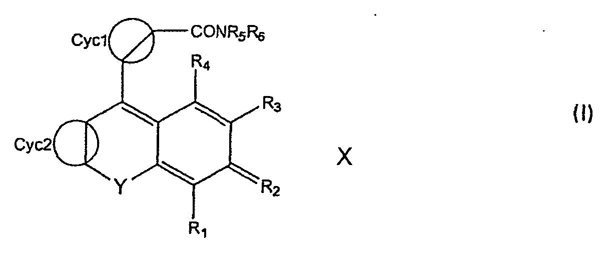 in which
Y=oxygen, sulfur, selenium, CRₐR_{b}, NR_{c}, a direct linkage or is -R₁₄ and -R₁₅;
R₁, R₃, R₄ are independently hydrogen, halogen, -O^{e}, a hydroxyl group, thiol group, amino group, ammonium group, sulfo group, phospho group, nitro group, carbonyl group, carboxyl group, a carboxylic acid derivative, a nitrile group, isonitrile group, cyanate group, isocyanate group, thiocyanate group, isothiocyanate group or a straight-chain, branched or cyclic saturated or unsaturated hydrocarbon group having up to 40 carbon atoms;
Rₐ, R_{b}, R_{c} and R₁₄, R₁₅ independently are as defined for R₁, R₃, R₄;
R₂ = O; or 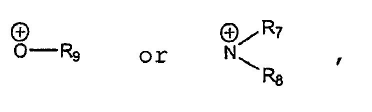
in which
R₇, R₈, R₉ independently are hydrogen or a straight-chain, branched or cyclic saturated hydrocarbon group which has up to 40 carbon atoms and which may contain heteroatoms and may be unsubstituted or substituted, or
R₁ together with R₂ is 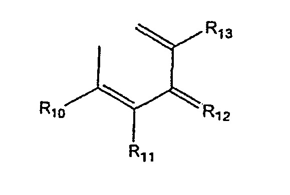 in which
R₁₀, R_{11,} R₁₃ are as defined for R₁, R₃, R₄;
R₁₂ = O, or 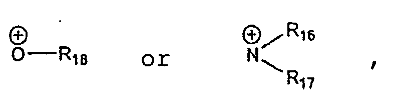 in which
R₁₆, R₁₇, R₁₈ are as defined for R₇, R₈, R₉;
R₅ is a straight-chain, branched or cyclic saturated or unsaturated hydrocarbon group having up to 40 carbon atoms, which may contain heteroatoms and may be unsubstituted or substituted;
R6 is a straight-chain or branched saturated or unsaturated hydrocarbon group which has up to 40 carbon atoms and which may contain heteroatoms and comprises a substituent selected from a carboxyl group, cyanate group, imino group, iminium group, isocyanate group, isothiocyanate group, nitro group, phospho group, sulpho group, thiocyanate group, thioether group, thiol group or a carboxylic acid derivative;
Cycl is an organic radical which comprises a ring system selected from aromatic, heteroaromatic, quinoidal and cycloaliphatic rings;
Cyc2 is an organic radical which comprises a ring system selected from aromatic, heteroaromatic, quinoidal and cycloaliphatic rings;
each of said radicals in the dye of the formula (I) being able to form a ring system with one or more neighboring radicals;
and X being one or more mono- or multivalent anions, when required for balancing the charge; with the proviso that
- Y = oxygen,
- Cycl = phenyl or substituted phenyl,
- Cyc2 = hydroxyl-, ether- or ester-substituted phenyl
and
- R₂ = 0
do not appear in the formula (I) at the same time.

2. Carboxamide-substituted dye according to Claim 1, in which Cyc2 is a nitrogen-containing heterocycle or a ring system substituted with at least one amino group
or/and R₂ = 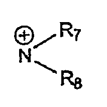 or,
together with R_{1,}= 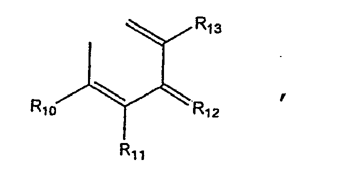 in which R₁₂ = 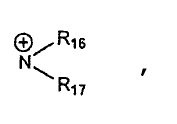 in which R₇, R₈; R₁₀, R₁₁, R₁₃ and R₁₆, R₁₇ are as defined in Claim 1.

3. Carboxamide-substituted dye according to Claim 1 or 2, in which Cyc2 in the formula (I) has a structure (A), (B), (C), (D), (E), (F), (G), (H) or (J), 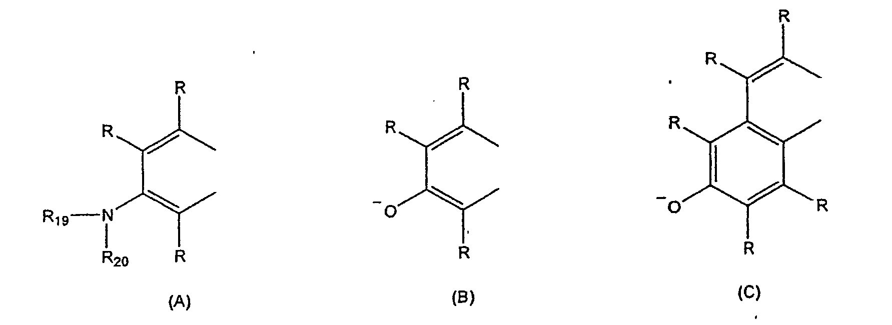 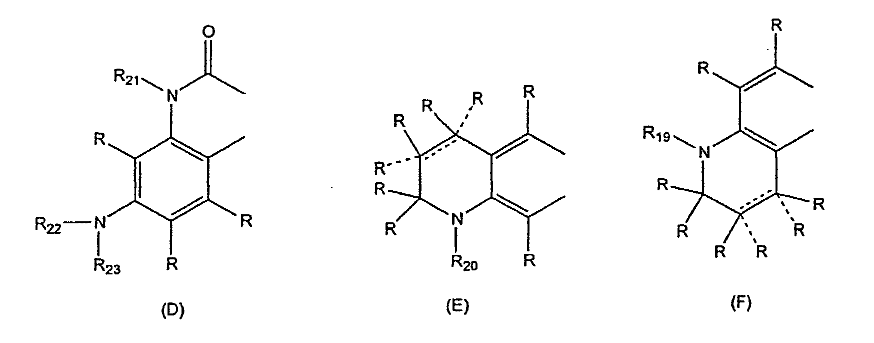 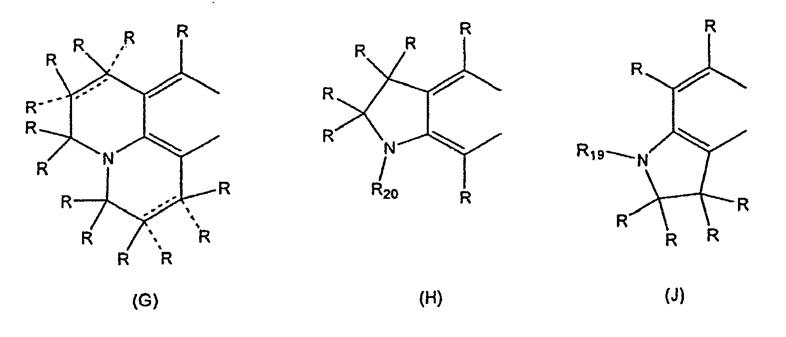
in which R in each case independently is defined as R₁, R₃, R₄ in Claim 1;
R₁₉, R₂₀ and R₂₂, R₂₃ are independently defined as R₇, R₈ in Claim 1; and
R₂₁ is defined as R₇ in Claim 1 and the dashed lines are, where appropriate, double bonds in the presence of which the radicals bound via a dashed line are absent.

4. Carboxamide-substituted dye according to any of the preceding claims, in which Cycl is substituted or unsubstituted phenyl, naphthyl, pyridyl or cyclohexyl.

5. Carboxamide-substituted dye according to any of the preceding claims, in which
R₂ = 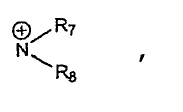 where R₇ and R₈ are as defined in Claim 1.

6. Carboxamide-substituted dye according to Claim 5, in which R₁ is bridged with R₈ or/and R₃ is bridged with R₇ and forms a ring system.

7. Carboxamide-substituted dye according to Claim 6, in which the ring system/s comprise(s) 5- or 6-membered rings.

8. Carboxamide-substituted dye according to Claim 7, in which a ring system of the structure (K), (L), (M), (N) or (0) is formed: 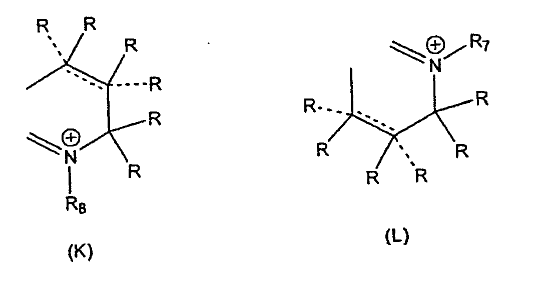 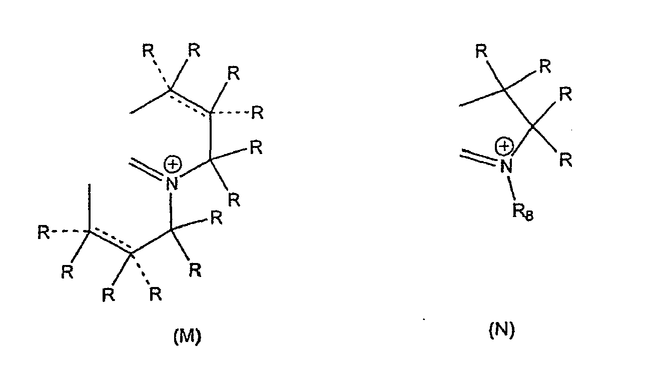 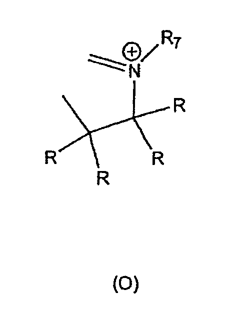 in which R in each case independently is defined as R₁, R₃, R₄ and R₇, R₈ are as defined in Claim 1, and the dashed lines are, where appropriate, double bonds, in the presence of which the radicals bound via a dashed line are absent.

9. Carboxamide-substituted dye according to any of Claims 1 to 4, in which
R₂ together with R₁ is 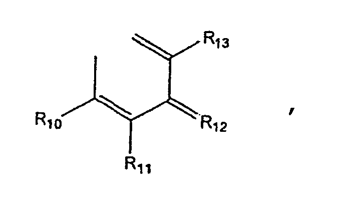 where R₁₀-R₁₃ are as defined in Claim 1.

10. Carboxamide-substituted dye according to Claim 9, in which
R₁₂ = O.

11. Carboxamide-substituted dye according to Claim 9, in which
R₁₂ = 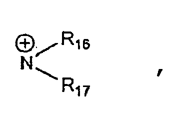 where R₁₆ and R₁₇ are as defined in Claim 1.

12. Carboxamide-substituted dye according to any of the preceding claims, in which Y = oxygen.

13. Carboxamide-substituted dye according to any of Claims 1 to 11, in which Y = sulfur, selenium or CRₐR_{b}, Rₐ and R_{b} being as defined in Claim 1.

14. Carboxamide-substituted dye according to any of Claims 1 to 11, in which Y = radicals -R₁₉ and -R₁₅, R₁₄ and R₁₅ being as defined in Claim 1.

15. Carboxamide-substituted dye according to Claim 8, in which Cyc1 is unsubstituted or substituted phenyl, Cyc2 has the structure (E) and Y = oxygen and R₇ and R₃ form a ring system (K), R₇ and R₃ being as defined in Claim 1.

16. Carboxamide-substituted dye according to Claim 8, in which Cyc1 is unsubstituted or substituted phenyl, Cyc2 has the structure (A) and Y = sulfur, selenium or CRₐR_{b}, Rₐ and R_{b} being as defined in Claim 1.

17. Process for preparing carboxamide-substituted dyes of the formula (I) according to any of Claims 1 to 16, comprising the following steps:
(a) converting the carboxyl group of a dye of the formula (II) 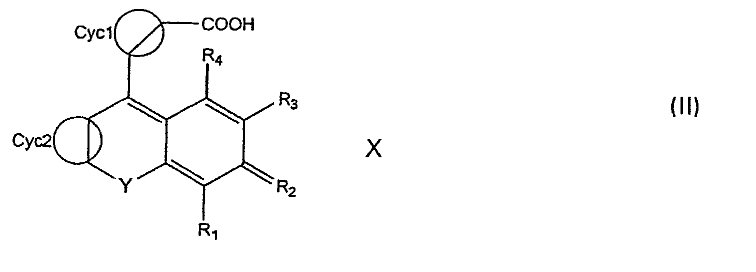 in which the radicals are defined as indicated in Claim 1, into an activated form;
(b) reacting the activated dye obtained in step (a) with a secondary amine HNR₅R₆; and
(c) isolating, if appropriate, the carboxamide-substituted dye of the formula (I) obtained in step (b).

18. Process according to Claim 17, in which step (a) is carried out at temperatures of from room temperature to 60°C.

19. Process according to Claim 17 or 18, in which an aprotic solvent is used in step (b).

20. Process according to any of Claims 17 to 19, in which N-hydroxysuccinimide, N-hydroxyphthalimide, N-hydroxynaphthalimide, O-(N-succinimidyl)-N,N,N',N'-tetramethyluronim tetrafluoroborate (TSTU) are used for activation.

21. Use of a carboxamide dye according to any of Claims 1 to 16 for qualitative or/and quantitative determination of an analyte.

22. Use according to Claim 21, in which the carboxamide-substituted dye of the formula (I) is coupled to the analyte to be detected or/and to a component of a detection reagent or/and to a support.

23. Use according to Claim 21 or 22, in which detection comprises an immunological detection or/and detection by way of nucleic acid hybridization.

24. Conjugate of a carboxamide-substituted dye of the formula (I) according to any of Claims 1 to 16 and a binding partner.

25. Conjugate according to Claim 24, in which the binding partner is selected from among peptides, polypeptides, nucleic acids, nucleosides, nucleotides, nucleic acid analogs and haptens.

26. Use of a conjugate according to Claim 24 or 25 in nucleic acid hybridization processes and immunochemical processes.

27. Use according to Claim 22 or 23, in which coupling to the analyte to be detected or/and the component of a detection reagent or/and the support takes place via the substituents R₅ or/and R₆ of the carboxamide-substituted dye of the formula (I), the radicals R₅ and R₆ being as defined in Claim 1.

28. Use according to Claim 27, in which coupling is carried out via a covalent bond.

## Revendications

1. Colorant substitué par un carboxamide de formule générale (I) : 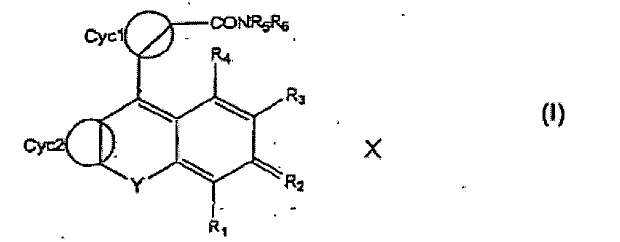 dans laquelle
Y = oxygène, soufre, sélénium, CRₐR_{b'} NR_{c'} une liaison directe ou -R₁₄ et -R₁₅ ;
R₁, R₃, R₄, indépendamment les uns des autres, représentent un atome d'hydrogène, d'halogène, -O^{θ}, un groupe hydroxy, un groupe thiol, un groupe amino, un groupe ammonium, un groupe sulfo, un groupe phospho, un groupe nitro, un groupe carbonyle, un groupe carboxy, un dérivé d'acide carboxylique, un groupe nitrile, un groupe isonitrile, un groupe cyanate, un groupe isocyanate, un groupe thiocyanate, un groupe isothiocyanate, ou un groupe hydrocarboné linéaire, ramifié ou cyclique, saturé ou insaturé, ayant jusqu'à 40 atomes de carbone ;
R_{a'} R_{b'} R_{c} et R_{14'} R_{15'} indépendamment les uns des autres, ont les mêmes significations que celles définies pour R₁, R₃, R₄ ;
R₂ = O ; ou 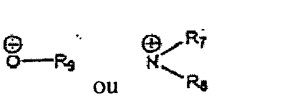 dans lesquelles
R₇, R₈, R₉, indépendamment les uns des autres, représentent un atome d'hydrogène ou un groupe hydrocarboné saturé linéaire, ramifié ou cyclique, ayant jusqu'à 40 atomes de carbone, qui peut comprendre des hétéroatomes et peut être substitué ou non substitué ou
R₁ conjointement avec R₂ représente 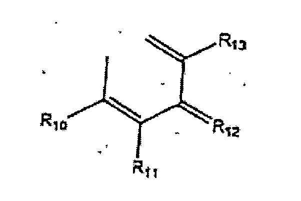 dans laquelle
R_{10'} R_{11'} R₁₃ ont les mêmes significations que celles définies pour R_{1'} R_{3'} R₄ ;
R₁₂ = O, ou 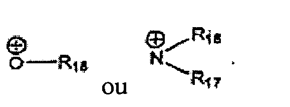
dans lesquelles R₁₆, R₁₇, R₁₈ ont les mêmes significations que celles définies pour R₇, R₈, R₉ ;
R₅ représente un groupe hydrocarboné linéaire, ramifié ou cyclique, saturé ou insaturé, ayant jusqu'à 40 atomes de carbone, qui peut contenir des hétéroatomes et peut être non substitué ou substitué ;
R₆ représente un groupe hydrocarboné linéaire ou ramifié, saturé ou insaturé, ayant jusqu'à 40 atomes de carbone, qui peut contenir des hétéroatomes et comprend un substituant choisi parmi un groupe carboxyle, un groupe cyanate, un groupe imino, un groupe iminium, un groupe isocyanate, un groupe isothiocyanate, un groupe nitro, un groupe phospho, un groupe sulfo, un groupe thiocyanate, un groupe thioéther, un groupe thiol ou un dérivé d'acide carboxylique ;
Cyc1 représente un radical organique, qui comprend un système cyclique choisi parmi les cycles aromatiques, hétéroaromatiques, quinoïdes et cycloaliphatiques ;
Cyc2 représente un radical organique qui comprend un système cyclique choisi parmi les cycles aromatiques, hétéroaromatiques, quinoïdes et cycloaliphatiques ; dans laquelle chacun des radicaux du colorant de formule (I) peut former un système cyclique avec un ou plusieurs des radicaux voisins ;
et X, quand cela est nécessaire pour l'équilibrage des charges, représente un ou plusieurs anions mono- ou polyvalents ;
à la condition que, dans la formule générale (I), les symboles suivants ne représentent pas simultanément
- Y = oxygène,
- Cyc1 = phényle ou phényle substitué,
- Cyc2 = phényle substitué par un hydroxy, un éther ou un ester
et
- R₂ = O.

2. Colorant substitué par un carboxamide selon la revendication 1, dans lequel Cyc2 représente un hétérocycle comprenant de l'azote ou un système cyclique, qui est substitué par au moins un groupe amino
et/ou R₂ = 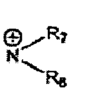 ou
conjointement avec R₁ = 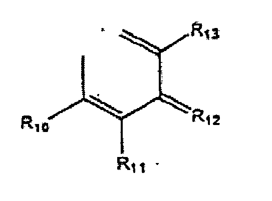 dans laquelle R₁₂ = 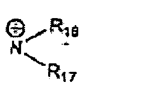 dans lesquelles R₇, R₈ R₁₀, R₁₁, R₁₃ et R₁₆, R₁₇ sont tels que définis dans la revendication 1.

3. Colorant substitué par un carboxamide selon la revendication 1 ou 2, dans lequel Cyc2 dans la formule (I) présente une structure (A), (B), (C), (D), (E), (F), (G), (H) ou (J) 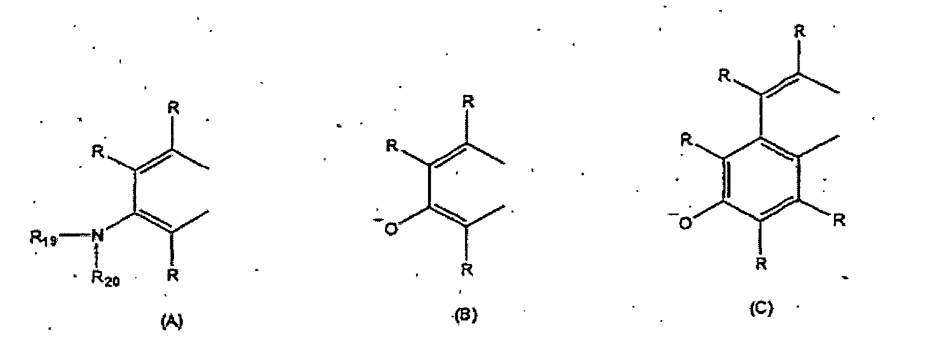 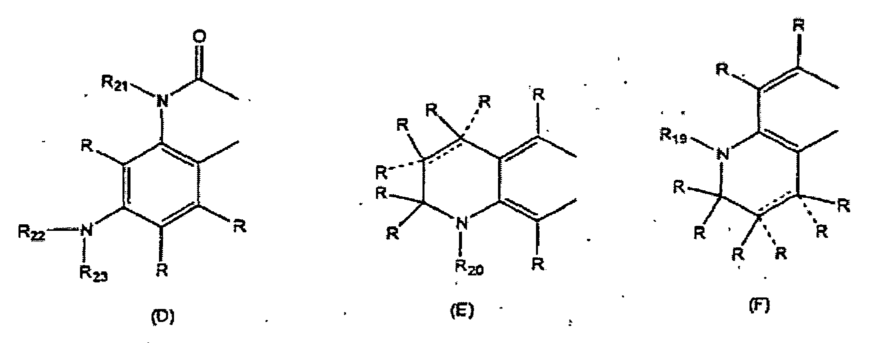 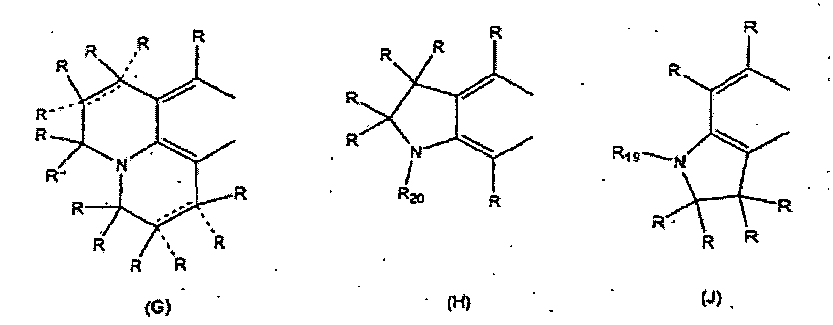
dans lesquelles R est défini, à chaque fois indépendamment, tel que R₁, R₃, R₄ dans la revendication 1 ;
R₁₉, R₂₀ et R₂₂, R₂₃, indépendamment, sont définis tels que R₇, R₈ dans la revendication 1 ;
et
R₂₁ est défini tel que R₇ dans la revendication 1 et les lignes en pointillés représentent le cas échéant des doubles liaisons, dont la présence annule les radicaux liés par une ligne en pointillés.

4. Colorant substitué par un carboxamide selon l'une des revendications précédentes, dans lequel Cyc1 représente un radical phényle, naphtyle, pyridyle ou cyclohexyle, substitué ou non substitué.

5. Colorant substitué par un carboxamide selon l'une des revendications précédentes, dans lequel
R₂ = 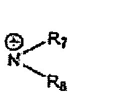 dans laquelle R₇ et R₈ sont tels que définis dans la revendication 1.

6. Colorant substitué par un carboxamide selon la revendication 5, dans lequel R₁ forme un pont avec R₈ et/ou R₃ avec R₇ et forme un système cyclique.

7. Colorant substitué par un carboxamide selon la revendication 6, dans lequel le ou les systèmes cycliques comprennent des cycles à 5 ou 6 chaînons.

8. Colorant substitué par un carboxamide selon la revendication 7, dans lequel un système cyclique de structure (K), (L), (M), (N) ou (O) est formé : 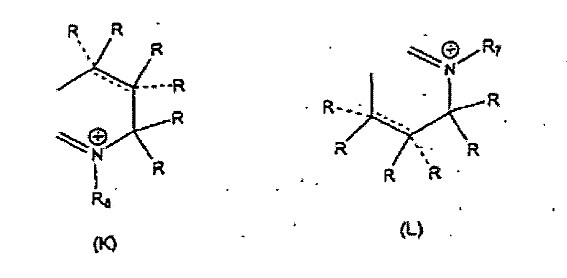 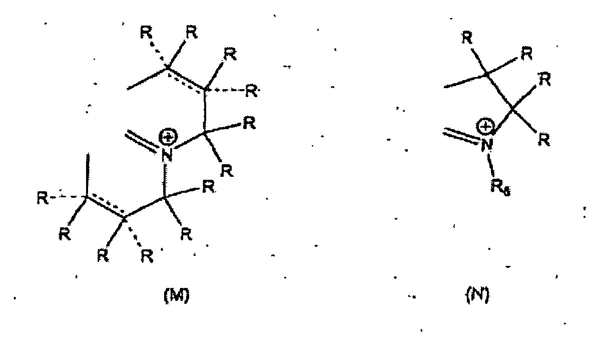 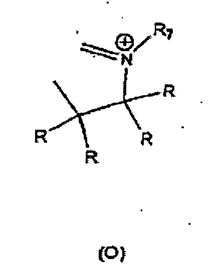 dans lesquels R est défini, à chaque fois indépendamment, tel que R₁, R₃, R₄ et R₇, R₈ dans la revendication 1,
et les lignes en pointillés représentent le cas échéant des doubles liaisons, dont la présence annule les radicaux liés par une ligne en pointillés.

9. Colorant substitué par un carboxamide selon l'une des revendications 1 à 4, dans lequel R₂ conjointement avec R₁ représente 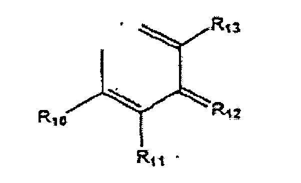 dans laquelle R₁₀ à R₁₃ sont tels que définis dans la revendication 1.

10. Colorant substitué par un carboxamide selon la revendication 9, dans lequel R₁₂ = O.

11. Colorant substitué par un carboxamide selon la revendication 9, dans lequel 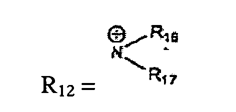 dans laquelle R₁₆ et R₁₇ sont tels que définis dans la revendication 1.

12. Colorant substitué par un carboxamide selon l'une des revendications précédentes, dans lequel Y = oxygène.

13. Colorant substitué par un carboxamide selon l'une des revendications 1 à 11, dans lequel Y = soufre, sélénium ou CRₐR_{b}, où Rₐ et R_{b} sont tels que définis dans la revendication 1.

14. Colorant substitué par un carboxamide selon l'une des revendications 1 à 11, dans lequel Y = les radicaux -R₁₄ et -R₁₅, où -R₁₄ et- R₁₅ sont tels que définis dans la revendication 1.

15. Colorant substitué par un carboxamide selon la revendication 8, dans lequel Cyc1 est un phényle le cas échéant substitué, Cyc2 présente la structure (E) et Y = oxygène et R₇ et R₃ forment un système cyclique (K), R₇ et R₃ sont tels que définis dans la revendication 1.

16. Colorant substitué par un carboxamide selon la revendication 8, dans lequel Cyc1 est un phényle le cas échéant substitué, Cyc2 présente la structure (A) et Y = soufre, sélénium ou CRₐR_{b}, où Rₐ et R_{b} sont tels que définis dans la revendication 1.

17. Procédé de préparation des colorants substitués par un carboxamide de formule générale (I) selon l'une des revendications 1 à 16, comprenant les étapes consistant à :
(a) transformer le groupe carboxyle d'un colorant de formule générale (II) 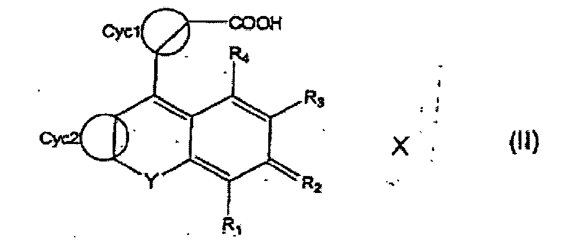 dans laquelle les radicaux sont définis tels qu'indiqué dans la revendication 1, en une forme activée ;
(b) faire réagir le colorant activé obtenu à l'étape (a) avec une amine secondaire HNR₅R₆ ; et
(c) le cas échéant, isoler le colorant substitué par un carboxamide de formule générale (I), obtenu à l'étape (b).

18. Procédé selon la revendication 17, dans lequel l'étape (a) est réalisée à des températures allant de la température ambiante à 60 °C.

19. Procédé selon la revendication 17 ou 18, dans lequel on utilise à l'étape (b) un solvant aprotique.

20. Procédé selon l'une des revendications 17 à 19, dans lequel on utilise pour l'activation le N-hydroxysuccinimide, le N-hydroxyphtalimide, le N-hydroxynaphtalimide, le tétrafluoroborate de O-(N-succinimidyl)-N,N,N',N'-tétraméthyluronium (TSTU).

21. Utilisation d'un colorant substitué par un carboxamide selon l'une des revendications 1 à 16 pour la détermination qualitative et/ou quantitative d'un analyte.

22. Utilisation selon la revendication 21, dans laquelle le colorant substitué par un carboxamide de formule générale (I) est couplé à l'analyte à détecter et/ou à un composant d'un réactif de détection et/ou à un support.

23. Utilisation selon la revendication 21 ou 22, dans laquelle la détection comprend une détection immunologique et/ou une détection par hybridation de l'acide nucléique.

24. Conjugué constitué d'un colorant substitué par un carboxamide de formule générale (I) selon l'une des revendications 1 à 16 et d'un partenaire de liaison.

25. Conjugué selon la revendication 24, dans lequel le partenaire de liaison est choisi parmi les peptides, les polypeptides, les acides nucléiques, les nucléosides, les nucléotides, les analogues d'acide nucléique et les haptènes.

26. Utilisation d'un conjugué selon la revendication 24 ou 25 dans des procédés d'hybridation de l'acide nucléique et des procédés immunochimiques.

27. Utilisation selon la revendication 22 ou 23, dans laquelle le couplage à l'analyte à détecter et/ou au composant d'un réactif de détection et/ou au support s'effectue par le biais des substituants R₅ et/ou R₆ du colorant substitué par un carboxamide de formule générale (I), dans laquelle les radicaux R₅ et R₆ sont tels que définis dans la revendication 1.

28. Utilisation selon la revendication 27, dans laquelle le couplage s'effectue par liaison covalente.
